(19) Europäisches Patentamt
European Patent Office
Office européen des brevets

(11) Veröffentlichungsnummer: **0 163 190**
**B1**

(12) # EUROPÄISCHE PATENTSCHRIFT

(45) Veröffentlichungstag der Patentschrift: **11.04.90**

(51) Int. Cl.[5]: **C 07 D 501/46,**
C 07 D 501/18, A 23 K 1/17

(21) Anmeldenummer: **85105841.2**

(22) Anmeldetag: **13.05.85**

(54) **Beta-Lactamantibiotika, Verfahren zu deren Herstellung sowie ihre Verwendung als Arzneimittel oder Wachstumsförderer in der Tieraufzucht oder als Antioxidantien.**

(30) Priorität: **22.05.84 DE 3419012**

(43) Veröffentlichungstag der Anmeldung:
**04.12.85 Patentblatt 85/49**

(45) Bekanntmachung des Hinweises auf die Patenterteilung:
**11.04.90 Patentblatt 90/15**

(84) Benannte Vertragsstaaten:
**AT BE CH DE FR GB IT LI NL SE**

(56) Entgegenhaltungen:
**EP-A-0 048 954**
**EP-A-0 049 448**
**EP-A-0 082 498**
**EP-A-0 098 433**
**EP-A-0 107 138**
**GB-A-2 076 801**

**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Angerbauer, Rolf, Dr.**
**Sterntalerweg 29**
**D-5600 Wuppertal 1 (DE)**
Erfinder: **Boberg, Michael, Dr.**
**Untere Bergerheide 47**
**D-5600 Wuppertal 1 (DE)**
Erfinder: **Metzger, Karl Georg, Dr.**
**Pahlkestrasse 75**
**D-5600 Wuppertal 1 (DE)**
Erfinder: **Zeiler, Hans-Joachim, Dr.**
**Elsbeekerstrasse 46**
**D-5690 Velbert 15 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 163 190 B1

**Beschreibung**

Die Erfindung betrifft β-Lactamverbindungen, Verfahren zu ihrer Herstellung sowie ihre Verwendung als Arzneimittel, insbesondere als antibaketerielle Mittel und ferner als Mittel zur Förderung des Wachstums und zur Verbesserung der Futterverwertung bei Tieren sowie Antioxidantien.

Cephalosporine, die als Acylseitenkette einen Aminothiazolylacrylsäurerest tragen, sind aus der EP—A—49 448 bekannt.

In der EP—A—0 017 138 werden antibiotische polare Cephemderivate beschrieben, die in 3-Stellung durch eine Pyridinomethylgruppe substituiert sind und in 7-Stellung eine 1-Aminothiazolyl-1-alkyliden-acetamidogruppe tragen. Außerdem ist die 1-Methyl-1-pyrolidinomethylgruppe als 3-Substituent in antimikrobiell wirksamen Cephemderivaten aus der DE—A—3 307 550 bekannt.

Die Erfindung stellt neue β-Lactamberbindungen zur Verfügung, die der allgemeinen Formel (I) entsprechen,

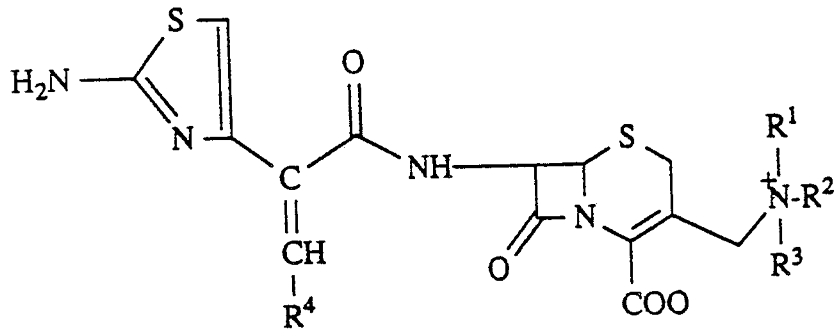

(I)

worin

$R^1$, $R^2$ und $R^3$ gleich oder verschieden voneinander sind und einen $C_1$—$C_6$-Alkylrest oder einen 3 bis 7 gliedrigen Ring darstellen, oder $R^1$ die obige Bedeutung hat und $R^2$ und $R^3$ gemeinsam mit dem $N^{\oplus}$-Atom einen 3 bis 7 gliedrigen Ring bilden, der gesättigt oder ungesättigt sein kann und ein oder zwei weitere Heteroatome enthalten kann, die Sauerstoff, Stickstoff und/oder Schwefel sein können, und

$R^4$ Wasserstoff, einen gegebenenfalls geradkettigen oder verzweigten Alkylrest mit bis zu 6 C-Atomen, der auch 1 oder 2 Doppelbindungen enthalten und und carbocyclisch sein kann, einen Arylrest der Formel

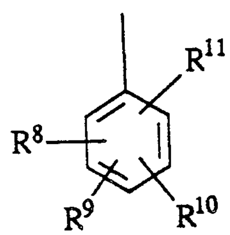

worin

$R^8$, $R^9$, $R^{10}$ und $R^{11}$ unabhängig voneinandere Wasserstoff, F, Cl, Br, $C_1$—$C_6$-Alkyl, Phenyl, eine Gruppe —$OCOR^{12}$, eine Gruppe

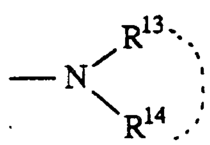

Nitro, Cyano, $C_1$—$C_4$-Alkoxy, $C_1$—$C_4$-Alkylthio, Hydroxycarbonyl, $C_1$—$C_4$-Alkoxycarbonyl, Aminocarbonyloxy, Hydroxycarbonyl-$C_1$—$C_6$-Alkyl, $C_1$—$C_4$-Alkoxycarbonyl-$C_1$—$C_6$-Alkyl, Sulfonyl oder Sulfo bedeutet, und

worin $R^{12}$ $C_1$—$C_6$-Alkyl bedeutet, und

worin $R^{13}$ und $R^{14}$ unabhängig voneinander Wasserstoff, oder gemeinsam oder unabhängig voneinander $C_1$—$C_6$-Alkyl oder $C_1$—$C_6$-Alkanoyl sein können.

einen 5- oder 6-Ring mit 1—4 Heteroatomen, gleich oder verschieden aus der Gruppe N, O und S, oder Hydroxycarbonyl, Hydroxycarbonyl-$C_1$—$C_4$-alkyl, $C_1$—$C_6$-Alkoxycarbonyl, Halogen oder Pseudohalogen bedeutet.

Die Bedeutung Alkyl umfaßt jeweils geradkettige oder verzweigte, gegebenenfalls substituierte Reste mit bis zu 6 C-Atomen, die auch ungesättigt, bevorzugt mit 1 oder 2 Doppelbindungen un carbocyclisch sein können.

In den Verbindungen der Formel (I) existiert zu jeder Strukturformel eine E- und eine Z-konfigurierte Verbindung gemäß der in J. Amer. Chem. Soc. *90*, 509 (1968) beschriebenen E/Z-Nomenklatur.

Bevorzugte Verbindungen der Formel I liegen in der Z-Konfiguration vor.

Wenn $R^1$ und/oder $R^2$ und/oder $R^3$ einen substituierten Alkylrest darstellen, dann bevorzugt einen $C_1$—$C_6$-Alkylrest mit vorzugsweise einem oder zwei Substituenten aus der Gruppe Hydroxy, Carboxy, $C_1$—$C_6$-Alkyloxycarbonyl, Formyl oder $C_1$—$C_6$-Alkylcarbonyl, Carbamoyl, Sulfo, Cyano, Nitro, Amino, Halogen, $C_1$—$C_6$-Alkyl- und Dialkylamino, $C_1$—$C_6$-Alkylcarbonylamino, $C_1$—$C_6$-Alkyloxy, $C_1$—$C_6$-Alkylthio, $C_1$—$C_6$-Alkylsulfinyl, $C_1$—$C_6$-Alkylsulfonyl, Phenyl, Naphthyl oder Pyridyl.

Wenn $R^1$ und/oder $R^2$ und/oder $R^3$ einen gesättigten oder ungesättigten gegebenenfalls substituierten 3- bzw 7-gliedrigen Ring darstellen, dann bevorzugt einen carbo- oder heterocyclischen Ring, der bis zu drei, bevorzugt ein- oder zwei Heteroatome enthalten kann, die Sauerstoff, Stickstoff und/oder Schwefel sein können.

Ist der carbo- oder heterocyclische Ring substituiert, dann bevorzugt mit einem oder zwei Substituenten aus der Gruppe $C_1$—$C_6$-Alkyl, Hydroxy, Hydroxy-$C_1$—$C_6$-Alkyl, Carboxy, $C_1$—$C_6$-Alkyloxycarbonyl, Formyl oder $C_1$—$C_6$-Alylcarbonyl, Carbamoyl, Sulfo, Cyano, Nitro, Amino, Halogen, $C_1$—$C_6$-Alkyl- und Dialkylamino, $C_1$—$C_6$-Alkylcarbonylamino, $C_1$—$C_6$-Alkyloxy, $C_1$—$C_6$-Alkylthio, $C_1$—$C_6$-Alkylsulfinyl oder $C_1$—$C_6$-Alkylsulfonyl.

Wenn $R^2$ und $R^3$ gemeinsam mit dem N-Atom einen gegebenenfalls substituierten heterocyclischen Ring bilden, dann bevorzugt einen 3- bis 7-gliedrigen Ring, der ein oder zwei Doppelbindungen und bis zu zwei weitere Heteroatome enthalten kann, die Sauerstoff, Stickstoff oder Schwefel sein können und an den ein weiterer 5- bis 6-gliedriger Ring ankondensiert sein kann.

Wenn der durch $R^2$ und $R^3$ gemeinsam mit dem N-Atom gebildete heterocyclische Ring substituiert ist, dann vorzugsweise mit einem oder zwei Substituenten, vorzugsweise gegebenenfalls substituiertes $C_1$—$C_6$-Alkyl, Hydroxy, Hydroxy-$C_1$—$C_6$-alkyl, Carboxy, $C_1$—$C_6$-Alkyloxycarbonyl, Formyl oder $C_1$—$C_6$-Alkylcarbonyl, deren Carbonylgruppen auch in ketalisierter Form vorliegen können, Carbamoyl, Sulfo, Cyano, Nitro, Amino, Halogen, $C_1$—$C_6$-Alkyl- und Dialkylamino, $C_1$—$C_6$-Alkylcarbonylamnino, $C_1$—$C_6$-Alkyloxy, $C_1$—$C_6$-Alkylthio, $C_1$—$C_6$-Alkylsulfinyl, $C_1$—$C_6$-Alkylsulfonyl, Aryl, Hetaryl oder Hetereocyclyl.

Wenn $R^4$ einen Alkylrest dartstellt, dann bevorzugt einen geradkettigen oder verzweigten, gegebenenfalls substituierten, Rest mit bis zu 6 C-Atomen. Alkyl im Rahmen dieser Definition umfaßt auch ungesättigte Reste vorzugsweise mit 1 oder 2 Doppelbindungen und carbocyclishe Reste.

Wenn der Alkylrest $R^4$ substituiert ist, dann vorzugsweise mit einem oder zwei Substituenten, vorzugsweise aus der Gruppe Halogen, vorzugswiese F, Cl oder Br, OH, Niedrigalkoxy, Oxo, Thio, Nitro, Cyano, Hydroxycarbonyl, Niedrigalkoxycarbonyl, Aminocarbonyloxy, Sulfo, Aryl, —O—COR$^5$,

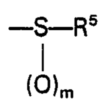 oder 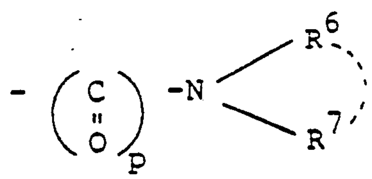

worin

m 0, 1 oder 2 bedeutet,

p 0 oder 1 bedeutet,

$R^5$ für Niedrigalkyl oder Aryl, vorzugsweise Phenyl steht und

$R^6$ und $R^7$ unabhängig voneinander für Wasserstoff oder gemeinsam oder einzeln für $C_1$—$C_6$-Alkyl oder $C_1$—$C_6$-alkanoyl stehen.

Die Bedeutung Alkyl umfaßt jeweils auch substituierte und/oder cyclische und/oder ungesättigte Strukturen.

Wenn $R^4$ einen heterocyclischen Rest darstellt, dann bevorzugt einen 5- oder 6-Ring, mit 1—4 Heteroatomen, gliech oder verschieden aus der Gruppe N, O und S. Ganz besonders bevorzugt sind die Pyridin-, Pyrimidin-, Pyrazin-, Pridazin, Furan-, Thiophen, Isoxazol- oder Thiazolreste.

Ganz besonders bevorzugt sind Verbindungen der Formel I in der Z-Konfiguration in denen

$R^1$, $R^2$ und $R^3$ gleich oder verschieden sind un einen $C_1$—$C_6$-Alkylrest, wie insbesondere Methyl, Ethyl, Propyl, Cyclopropyl, Cyclopropylmethyl, Cyclopentyl, Cyclopentylmethyl, oder einen substituierten $C_1$—$C_6$-Alkylrest, wie insbesondere Hydroxymethyl, Hydroxyethyl, Hydroxypropyl, Aminomethyl, Aminoethyl, Hydroxycarbonylmethyl, Hydroxycarbonylethyl, Cyanomethyl, Nitromethyl, Nitroethyl, Methoxymethyl, Methoxycarbonylmethyl, Trifluoromethyl darstellen,

oder in der

$R^1$ die obige Bedeutung hat und

$R^2$ und $R^3$ gemeinsam mit dem N-Atom einen 5- oder 6-gliedrigen heterocyclischen Ring bilden, der ein weiteres Heteroatom enthalten kann und an den ein weiterer Ring ankondensiert sein kann wie insbesondere Pyrrolidinium, Piperidinium, Morpholinium, Pyrrolinium, Pyrazolidinium, Indolinium, Isoindolinium, Oxazolidinium, Thiazolidinium, Thiomorpholinium und der gegebenenfalls substituiert sein kann durch $C_1$—$C_6$-Alkyl, wie insbesondere Methyl, Ethyl, Propyl das wiederum substituierte sein kann, z.B. durch Hydroxy, Carboxy, Cyano, Nitrol, Amino, Halogen, Alkoxy, durch $C_1$—$C_6$-Alkoxycarbonyl, wie Methoxycarbonyl, Formyl oder $C_1$—$C_6$-Alkylcarbonyl, wie insbesondere Methyl- und Ethylcarbonyl, durch Carbamoyl, Sulfo, Cyano, Nitro, Halgoen, wie insbesondere Fluorid und Chlorid, Amino, $C_1$—$C_6$-Alkyl- und Dialkylamino, wie insbesondere Methylamino und Diethylamino, $C_1$—$C_6$-Alkylcarbonylamino,

wie insbesondere Methylcarbonylamino und Ethylcarbonylamino, $C_1$—$C_6$-Alkyloxy, wie insbesondere Methoxy, $C_1$—$C_6$-Alkylthio, wie insbesondere Methylthio, $C_1$—$C_6$-Alkylsulfinyl, wie insbesondere Methylsulfinyl, $C_1$—$C_6$-Alkylsulfonyl, wie insbesondere Methyl- und Ethylsulfonyl, durch Aryl wie insbesondere Phenyl oder Naphthyl, die auch substituiert sein können, durch Hetaryl, wie Pyridyl, das auch substituiert sein kann und

$R^4$ einen Niedrigalkylrest, wie Methyl, Ethyl, Propyl, Cyclopropyl, Cyclopentyl, einen unsubstituierten oder substituierten Arylrest, wie Phenyl, Dichlorphenyl, Trichlorphenyl, Hydroxycarbonylphenyl, Hydroxycarbonyl-$C_1$—$C_6$-alkylphenyl, einen heterocyclischen 5- oder 6-Ring, wie Pyridyl oder Aminothiazolyl, Hydroxycarbonyl, Hydroxycarbonyl-$C_1$—$C_4$-alkyl, wie 1-Hydroxycarbonyl-1-methylethyl, Niedrigalkoxycarbonyl, wie Methoxycarbonyl oder $C_1$—$C_4$-Alkylsulfonyl, wie Methylsulfonyl bedeutet.

Die Erfindung stellt ferner neueβ-Lactamverbindungen der allgemeinen Formel IV, in der $R^1$, $R^2$, $R^3$ die gleiche Bedeutung wie in Formel I besitzen, zur Verfügung, die als Zwischenprodukte zur Herstellung der Verbindungen der Formel I verwendet werden können.

Die Verbindungen der allgemeinen Formel I können dadurch erhalten werden, daß Verbindungen der allgemeinen Formel (III)

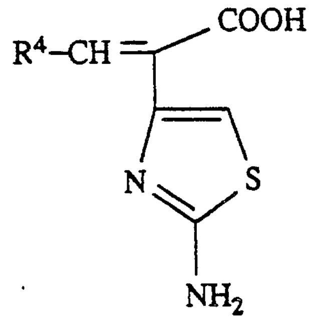

(III)

worin

$R^4$ die oben angegebene Bedeutung hat, in denen die Aminogruppe geschützt oder ungeschützt vorliegen kann, nach Aktivierung der Carboxylgruppe durch Überführung in ein gemischtes Anhydrid, beispielsweise mit Chlorameisensäureethylester oder Methansulfonsäurechlorid, nach überführung in das Säurehalogenid oder nach Überführung in einen aktivierten Ester mit beispielsweise N-Hydroxybenztriazol und Dicyclohexylcarbodiimid, mit Verbindungen der allgemeinen Formel (IV),

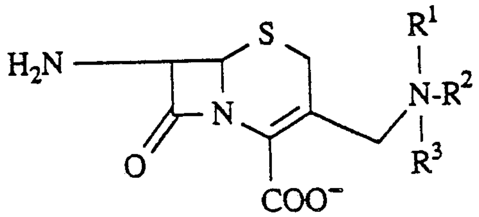

(IV)

worin $R^1$, $R^2$ und $R^3$ die obengenannte Bedeutung besitzen, zur Umsetzung gebracht werden, dann gegebenenfalls Schutzgruppen abgespalten werden und die gewünschten Salze oder aus Salzen die freien Säuren hergestellt werden.

Für die Kupplung von Carbonsäuren (III) an β-Lactame der Formel IV kann eine große Zahl von aus der Cephalosporin- bzw. Penicillinchemie bekannten Methoden verwendet werden. Es hat sich als vorteilhaft erwiesen, die Carbonsäuren der allgemeinen Formel III ohne Amin-Schutzgruppe zu aktivieren und dann mit den β-Lactamen der Formel IV, die als Salze mit einem Amin in Lösung gebracht wurden, zu kuppeln. Besonders vorteilhaft ist die Aktivierung mit Sulfonsäurederivaten der Formel (V) zu Anhydriden der Formel VI

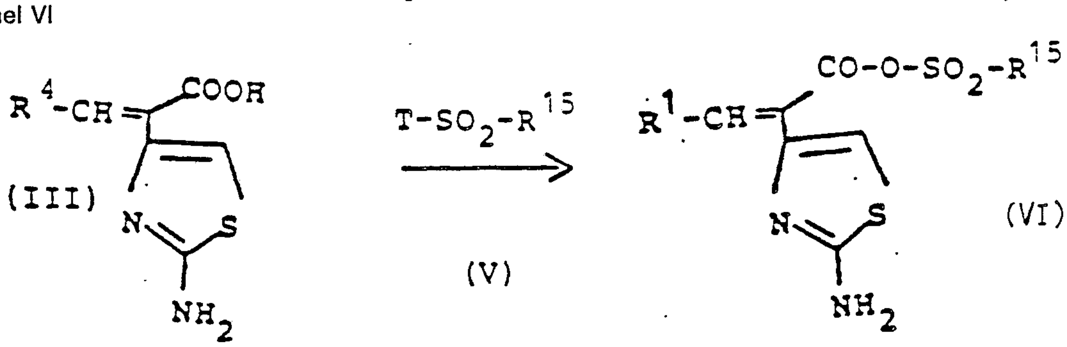


(III) (V) (VI)

worin

T einen Rest $R^{15}$—$SO_2O$ oder Halogen darstellt und

$R^{15}$ einen Alkyrest mit 1—10 C-Atomen bedeutet, der gegebenenfalls durch Fluor, Chlor, CN, Phenyl, Alkyloxycarbonyl, Alkyloxy oder Alkyl substituiert sein kann, wobei letztere Alkylreste 1—4 C-Atome tragen können, oder ein Phenylrest, der gegebenenfalls durch Fluor, Chlor, Brom, CN, Alkyl, Alkyloxy, Alkylthio, Alkylcarbonyl- wobei letztere Alkylgruppen 1—4 C-Atomen tragen können — Nitro, Trifluormethyl und Phenyl substituierte sein kann.

Wenn $R^{15}$ substituiert ist, sind vorzugsweise 1—3 Substituenten, vorzugsweise die genannten vorhanden.

Ganz besonders bevorzugt stellt $R^{15}$ einen Methyl- oder p-Tolylrest dar.

Die gemischten Anhydride der Formel VI werden hergestellt, indem man die Carbonsäuren der Formel III und 1-1,4 Äquivalente eines Amins in einem Lösungsmittel löst und mit 1 bis 1,2 Äquivalenten eines Sulfonsäurederivates der Formel V reagieren läßt.

Als Lösungsmittel eignen sich alle Solventien, die unter den Reaktionsbedingungen stabil sind, wie z.B. Diethylether, Tetrahydrofuran, Acetonitril, Aceton, Methylenchlorid, Chloroform, oder Dimethylformamid.

Als Amin eignen sich tertiäre Amine wie z.B. Triethylamin oder Tributylamin, aber auch sterisch gehinderte sekundäre Amine, wie z.B. Diisopropylamin.

Die Umsetzungen können bei Temperaturen zwischen −80°C und Raumtemperatur durchgeführt werden, wobei tiefe Temperaturen eine Isomerisierung der Substituenten an der Doppelbindung vermeiden. Vorteilhaft wird die Aktivierung Mit $Cl—SO_2CH_3$, in Dimethylformamid bei −40 bis −60°C innerhalb von 0,2 bis 24 Stunden, vorzugsweise 0,5 bis 5 Stunden, durchgeführt.

Zum Lösen der Verbindungen der Formel IV können die bei der Herstellung der Verbindungen der Formel VI genannten Lösungsmittel sowie als Base die dort genannten Amine verwendet werden.

Besonders vorteilhaft ist auch eine Aktivierung der Carbonsäuren der allgemeinen Formel III durch Überführung in einen aktivierten Ester mit beispielsweise N-Hydroxysuccinimid und Dicyclohexylcarbodiimid oder 1-Hydroxybenztriazol und Dicyclohexylcarbodiimid.

Als Lösungsmittel eignen sich alle Solventien, die sich auch für die Herstellung von Anhydriden der Formel VI eignen.

Die Umsetzungen können bei Temperaturen zwischen −30° und +100° durchgeführt werden. Vorteilhaft wird mit 1-Hydroxybenztriazol und Dicyclohexylcarbodiimid in Dimethylformamid bei Raumtemperatur 2 bis 6 Stunden aktiviert, dann von ausgefallenen Dicyclohexylharnstoff abgesaugt und mit einer Verbindung der Formel IV in Form einer Lösung ihres Aminsalzes innerhalb von 2 bis 24 Studen umgesetzt. Zum Lösen der Verbindungen der Formel IV können die bei der Herstellung der Verbindungen der Formel VI genannten Lösungsmittel sowie als Base die dort genannten Amine verwendet werden.

Die Verbindungen der Formel IV erhält man, indem aus Verbindungen der Formel VII die Aminschutzgruppe $R^{16}$ abspaltet. Dabei kann $R^{16}$ entweder eine säurelabile Schutzgruppe

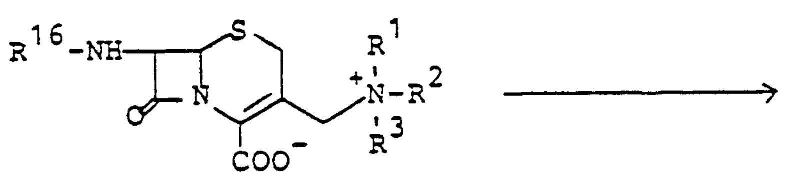

IV

VII

wie die t-Butyloxycarbonylgruppe oder vorteilhaft eine enzymatisch abspaltbare Schutzgruppe sein. Bevorzugte enzymatisch abspaltbare Schutzgruppen sind Phenacetyl oder 2-Thienylacetyl. Die enzymatische Abspaltung erfolgt bei Raumtemperatur in Wasser oder einem Gemisch aus Wasser und einem polaren organischen Lösungsmittel wie zum Beispiel Acetonitril oder Tetrahydrofuran mit immobilisierter Penicillin-G-Acylase bei pH 7—8, bevorzugt bei pH 7,5—7,8. Während der Abspaltung wird der pH-Wert durch Zugabe einer Base wie Lithiumhydroxid, Natriumhydroxid, Kaliumhydroxid, oder eines tert. Amins zum Beispiel Triethylamin, Tripropylamin, Tributylamin, Pyridin konstant gehalten.

Die Verbindungen der Formel VII lassen sich aus Estern der Formel VIII über Zwischenverbindungen der Formel IX herstellen.

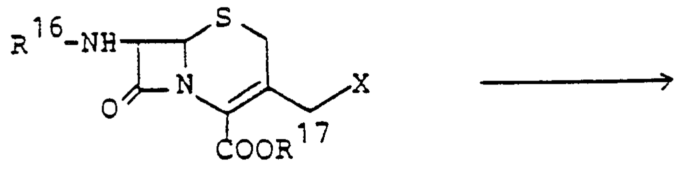

VIII

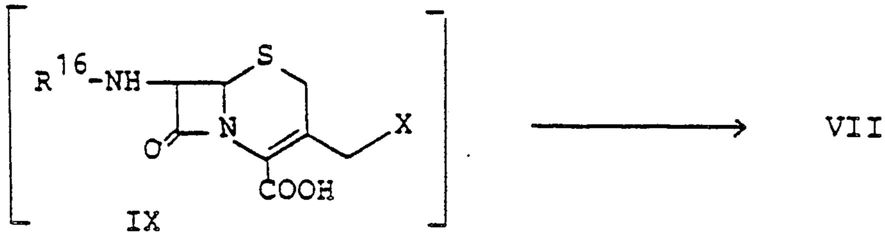

IX

VII

In den Estern der Formel VIII stellt X eine Fluchtgruppe, wie Mesylat, Tosylat, Brosylat, Triflat, Nonaflat, Jodid, Bromid, Chlorid, und $R^{17}$ eine in der Cephalosporinchemie übliche Säureschutzgruppe, bevorzugt eine sauer abspaltbare Schutzgruppe, wie. z.B. Benzhydryl, 4-Methoxydiphenylmethyl, t-Butyl, dar.

Die Vebindungen der Formel VIII werden duch Abspaltung der Säureschutzgruppe $R^{17}$ an die reaktiven freien Säuren der Formel IX überführt. Bei den bevorzugten säurelabilen Schutzgruppen $R^{17}$ wird die Schutzgruppen in einem organischen Lösungsmittel abgespalten. Bevorzugt ist die Abspaltung der Benzhydrylschutzgruppe in Methylenchlorid mit Trifluoressigsäure möglicherweise unter Zusatz eines Alkoxybenzols, bevorzugt Methoxybenzol. Die Abspaltung erfolgt bei −20°C bis +30°C, bevorzugt bei 0°C innerhalb von 5 Minuten bis einer Stunde, bevorzugt innerhalb von 20 Minuten.

Die Säure der Formel IX kann nach Abspaltung der Schutzgruppe isoliert werden. Vorteilhaft wird jedoch nicht isoliert, sondern direkt und ohne Reinigung zu Verbindungen der Formel VII umgesetzt. Dazu wird die bei der Reaktion VIII → IX entstehende Lösung von IX schonend im Vakuum eingeengt. Die zurückbleibende rohe Säure wird in einem organischen Lösungsmittel, bevorzugt in Tetrahydrofuran, aufgenommen und mit 2—20 Äquivalenten, bevorzugt mit 5—10 Äquivalenten, eines tert. Amins der Formel $NR_1R_2R_3$ wobei $R^1$, $R^2$, $R^3$ die vorstehend genannte. Bedeutung haben, zu Verbindungen der Formel VII umgesetzt. Die Umsetzung wird bei Temperaturen zwischen −20°C und 40°C, bevorzugt bei 25°C, innerhalb von 10 Minuten bis zwei Stunden, bevorzugt innerhalb von 30 Minuten, durchgeführt. Das Produkt kann nach Beendigung der Reaktion durch Zugabe von Diethylether ausgefällt werden. Das so erhaltene Rohprodukt kann an einem Harz wie Diaion HP 20 oder XAD 7 gereinigt werden. Es ist auch vorteilhaft möglich, das Rohprodukt direkt zu Verbindungen der Formel IV weiter umzusetzen.

Die Verbindungen der Formel VII lassen sich alternativ auch aus Säuren der Formel X herstellen, in denen $R^{16}$ die vorstehend.

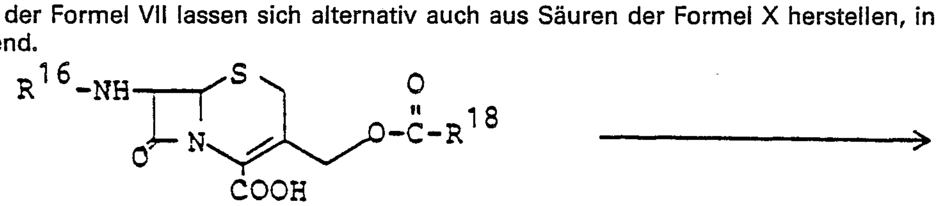

X

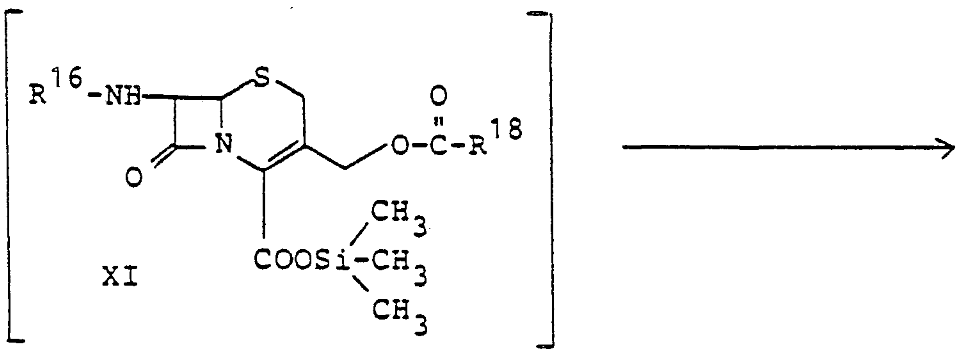

XI

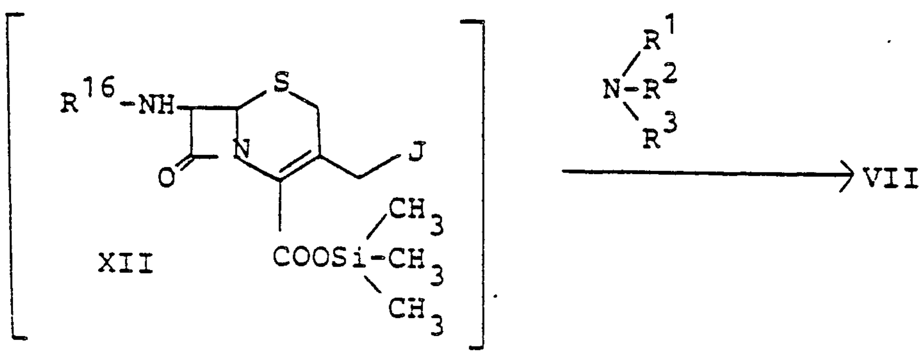

XII

VII

genannte Bedeutung hat und $R^{18}$ ein gegebenenfalls substituiertes Alkyl oder Aryl darstellt, wie Methyl, Ethyl, Propyl, Chlormethyl, Dichlormethyl, Trichlormethyl, Trifluormethyl, Phenyl. Ganz besonders bevorzugt stellt $R^{18}$ eine Methylgruppe dar.

Die Ausgangsverbindungen der Formel X werden in einem geeignten organischen Lösungsmittel suspendiert und durch Silylierung zum Silylester XI in Lösung gebracht. Besonders geeignete organische Lösungsmittel sind Chloroform, Methylenchlorid, Dichlorethan. Die Silylierung wird mit einem üblichen Silylierungsmittel wie Trimethylchlorsilan (TMCS), Hexamethyldisilazan (HMDS), NO-Bis(trimethylsilyl)-acetamid (BSA), N,O-Bis(trimethylsilyl)trifluoracetamid (BSTFA), N-Methyl-N-trimethylsilylacetamid (MSA), N-Methyl-N-trimethylsilyl-trifluoroacetamid (MSTFA), 1,3-Bis(trimethylsilyl)harnstoff, Trimethylsilyltrifluoromethansulfonat durchgeführt. Dabei können auch mehrere Silylierungsmittel im Gemisch eingesetzt werden.

Die Silylierung erfolgt bei −30°C bis +70°C, bevorzugt bei −10°C bis +10°C, innerhalb von 5 Minuten bis 30 Minuten. Vorteilhaft wird ein bis zu zehnfacher Überschuß des Silylierungsmittels eingesetzt, bevorzugt ein zweibis fünffacher Überschuß.

Die so erhaltene Lösung des Trimethylsilylesters der Formel XI wird bei −40°C bis +30°C, bevorzugt bei −10°C bis +10°C, mit ein bis zehn Äquivalenten, bevorzugt mit drei bis vier Äquivalenten eines Trialkylsilyljodids, besonders bevorzugt Trimethylsilyljodid, innerhalb von 15 Minuten bis 2 Stunden, bevorzugt innerhalb von 30 Minuten bis 1 Stunde, zu Verbindungen der Formel XII umgesetzt.

Die Verbindungen der Formel XII werden vorteilhaft nicht isoliert, sondern direkt ohne Reinigung mit Aminen

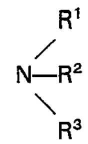

zu den Verbindungen der Formel VII umgesetzt.

Die Verbindungen der allgemeinen Formel I können alternativ auch dadurch hergestellt werden, daß Verbindungen der Formel XIII,

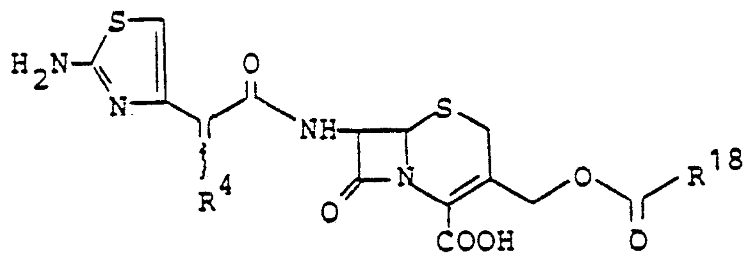

XIII

in denen $R^4$ und $R^{18}$ die obengenannte Bedeutung haben, analog wie vorher für die Umsetzung von Verbindungen der Formel X zu Verbindungen der Formel VII beschrieben, direkt ohne Isolierung der Zwischenstufen nach Silylierung und überführung ins Jodid mit Aminen

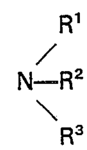

zu Verbindungen der Formel I umgesetzt werden.

Die erfindungsgemäßen Verbindungen weisen eine starke und breite antimikrobielle Wirksamkeit besonders gegen gramnegative und grampositive Bakterien auf. Diese Eigenschaften ermöglichen ihre Verwendung als chemotherapeutische Wirkstoffe in der Medizin. Mit ihrer Hilfe können die durch gramnegative und gram-positive Bakterien und bakterienähnliche Mikroorganismen hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen un systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z.B. Staphylococcus aureus, Staph. epidermidis, Staph. aerogenes und Graffkya tetragena (Staph. = Staphylococcus);

Lactobacteriaceae, wie Streptokokken, z.B. Streptococcus pyogenes, α- bzw. β-hämolysierende Streptokokken, nicht (γ-)-hämolysierende Streptokokken, Str. viridans, Str. faecalis (Enterokokken) und Dipolococcus pneumoniae (Pneumokokken) (Str. = Streptooccus);

Enterobacteriaceae, wie Escherichia -Bakterien der Coli-Gruppe: Escherichia-Bakterien, z.B. Escherichia coli, Enterobacter-Bakterien, z.B. E. aerogenes, E. cloacae, Klebsiella-Bakterien, z.B. K. pneumoniae, Serratia, z.B. Serratia marcescens (E. = Enterobacter) (K. = Klebsiella), Proteae-Bakterien der Proteus-Gruppe: z.B. Proteus vulgaris, Pr. morganii, Pr. rettgeri, Pr. mirabilis, (Pr. = Proteus);

Pseudomonadaceae, wie Pseudomonas-Bakterien, z.B. Pseudomonas aeruginosa, (Ps = Pseudomonas);

Bacteroidaceae, wie Bacteroides-Bakterien, z.B. Bacteroides fragilis, (B. = Bacteroides).

Die obige Aufzählung von Erregern ist lediglich beispielhaft und keineswegs beschränkend aufzufassen.

Als Krankheiten, die durch die erfindungsgemäßen Verbindungen verhindert, gebessert und/oder geheilt werden können, seien beispielsweise genannt:

Erkrankungen der Atemwege und des Rachenraumes;

Otitis; Pharyngitis; Pneumonie; Peritonitis; Pyelonephritis; Cystitis, Endocarditis; Systeminfektionen; Bronchitis; Arthritis; lokale Infektionen.

Zur vorliegenden Erfindung gehören pharmazeutische Zubereitungen, die neben nichttoxischen, inerten pharmazeutisch geeigneten Trägerstoffen einen oder mehrere erfindungsgemäße Verbindungen enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einen Bruchteil oder einem Vielfachen einer Einzeldosis entsprechen. Die Dosierungseinheiten können z.B. 1, 2, 3, oder Einzeldosen oder ½, ⅓ oder ¼ einer Einzeldosis enthalten. Eine Einzeldosis enthält vorzugsweise die Menge Wirkstoff, dei bei einer Applikation verabreicht wird und die gewöhnlich einer ganzen, einer halben oder einem Drittel oder einem Viertel einer Tagesdosis entspricht.

Unter nichttoxischen inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seine Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calcium-carbonat und Natriumcarbonat, (e) Lösungsverzögerer, z.B. Paraffin und (f) Resorptionsbeschleuniger, z.B. quaternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calciummagnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmitteln enthaltenen Überzügen und Hüllen versehen sein und auch so zusammengesetzt sein, daß sie den oder die Wirkstoffe nur oder bevorzugt in einem bestimmten Teil des Intestinaltraktes gegebenenfalls vorzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett un höhere Ester (z.B. $C_{14}$-Alkohol mit $C_{16}$-Fettsäure) oder Gemische dieser Stoffe.

Zur parenteralen Applikation können die Lösungen auch in steriler und blutisotonischer Form vorliegen.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% oder Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitonal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemäßen, Wirkstoffe in Gesamtmengen von etwa 1 bis etwa 1000, vorzugsweise 1 bis 200 mg/kg Körpergewicht je 24 Stunden, gegebenenfalls in Form mehrerer

Einzelgaben, zur Erzielung der gewünschten Ergebnisse zu verabreichen. Eine Einzelgabe enthält den oder die erfindungsgemäßen Wirkstoffe, vorzugsweise in Mengen von etwa 1 bis etwa 250, insbesondere 1 bis 60 mg/kg Körpergewicht Es kann jedoch erforderlich sein, von den genannten Dosierungen abzuweichen, und zwar in Abhängigkeit von der Art und dem Körpergewicht des zu behandelnden Objekts, der Art und der Schwere der Erkrankungen, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die erfindungsgemaßen Verbindungen können zum Zwecke der Erweiterung des Wirkungsspektrums mit einem anderen β-Lactamantibiotikum oder auch mit Aminoglykosidantibiotika, wie z.B. Gentamicin, Sisomicin, Kanamicin, Amikacin oder Tobramicin kombiniert werden.

Die erfindungsgemäßen Wirkstoffe können in allen Bereichen der Tierzucht als Mittel zur Förderung und Beschleunigung des Wachstums und zur Verbesserung der Futterverwertung bei gesunden und kranken Tieren verwendet werden.

Die Wirksamkeit der Wirkstoffe ist hierbei weitgehend unabhängig von der Art und dem Geschlecht weitgehend der Tiere. Besonders wertvoll erweisen sich die Wirkstoffe bei der Aufzucht und Haltung von Jung- und Masttieren. Als Tiere, bei denen die Wirkstoffe zur Förderung und Beschleunigung des Wachstums und zur Verbesserung der Futterverwertung eingesetzt werden können, seien beispielsweise folgende Nutz- und Ziertiere genannt:

Warmblüter wie Rinder, Schweine, Pferde, Schafe, Ziegen, Katzen, Hunde, Kaninchen; Pelztiere, z.B. Nerze, und Chinchilla; Geflügel, z.B. Hühner, Gänse, Enten, Truthähne, Tauben, Papageien und Kanarienvögel und Kaltblüter wie Fische, z.B. Karpfen und Reptilien, z.B. Schlangen.

Die Mengen der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,01 bis 50, insbesondere 0,1 bis 10 mg/kg Körpergewicht pro Tag. Die Dauer der Verabreichung kann von wenigen Stunden oder Tagen bis zu mehreren Jahren betragen. Die zu verabreichende Menge des Wirkstoffs sowie die entsprechende Dauer der Verabreichung hängen insbesondere von der Art, dem Alter, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Gesundheitszustand der Tiere ab. So kann die Verabreichung einmal oder mehrmals täglich in regelmäßigen oder unregelmäßigen Abständen oral oder parenteral erfolgen. Aus Zweckmäßigkeitsgründen ist in den meisten Fällen eine orale Verabreichung, insbesondere im Rhythmus der Nahrungs- und/oder Getränkeaufname der Tiere, vorzuziehen. Unter Nahrung im Sinne der vorliegenden Erfindung werden sowohl feste als auch flüssige Nahrung und auch Getränke und Wasser verstanden.

Die Wirkstoffe können als reine Stoffe oder in formulierter Form, also in Mischung mit nichttoxischen inerten Trägerstoffen beliebiger Art, z.B. mit Trägerstoffen und in Formulierungen, wie sie bei nutritriven Zubereitungen üblich sind, verabreicht werden.

Die Wirkstoffe werden gegebenenfalls in formulierter Form zusammen mit pharmazeutischen Wirkstoffen, Mineralsalzen, Spurenelementen, Vitaminen, Eiweißstoffen, Fetten, Farbstoffen und/oder Geschmacksstoffen in geeigneter Form verabreicht.

Empfehlenswert ist die orale Verabreichung zusammen mit dem Futter und/oder Trinkwasser, wobei je nach Bedarf der Wirkstoff der Gesamtmenge oder nur Teilen des Futters und/oder des Trinkwassers zugegeben wird.

Die Wirkstoffe werden nach üblichen Methoden durch einfaches Mischen als reine Stoffmischung; vorzugsweise in fein verteilter Form oder in formulierter Form in Mischung mit eßbaren nichttoxischen Trägerstoffen, gegebenenfalls in Form eines Praemix oder eines Futterkonzentrates, dem Futter und/oder Trinkwasser beigefügt ist, hergestellt.

Das Futter und/oder Trinkwasser kann beispielsweise die Virkstoffe in einer Gewichtskonzentration von etwa 0,01 bis 50, insbesondere 0,1 bis 10 ppm enthalten. Die optimale Höhe der Konzentration der Wirkstoffe in dem Futter und/oder Trinkwasser ist insbesondere abhängig von der Menge der Futter- und/oder Trinkwasseraufnahme der Tiere und kann durch jeden Fachmann leicht ermittelt werden.

Die Art des Futters und seine Zusammensetzung ist hierbei ohne Belang. Es können alle gebräuchlichen oder speziellen Futterzusammensetzungen verwendet werden, die vorzugsweise das übliche, für eine ausgewogene Ernährung notwendige Gleichgewicht aus Energie- und Aufbaustoffen einschließlich Vitaminen und Mineral stoffen enthalten. Das Futter kann sich beispielsweise zusammensetzen auf pflanzlichen Stoffen, z.B. Heu, Rüben, Getreide, Getreidenebenprodukten, tierischen Stoffen, z.B. Fleisch, Fetten, Konchenmehl, Fischprodukten, Vitaminen, z.B. Vitamin A, D-Komplex und B-Komplex, Proteinen, Aminosäuren, z.B. DL-Methionin und anorganischen Stoffen, z.B. Kalk und Kochsalz.

Futterkonzentrate enthalten die Wirkstoffe neben eßbaren Stoffen, z.B. Roggenmehl, Maismehl, Sojobohnenmehl oder Kalk, gegebenenfalls mit weiteren Nähr- und Aufbaustoffen sowie Proteinen, Mineralsalzen und Vitaminen. Sie können nach den üblichen Mischmethoden hergestellt werden.

Vorzugsweise in Praemixen und Futterkonzentraten können die Wirkstoffe gegebenenfalls auch durch ihre Oberfläche bedeckende geeignete Mittel, z.B. mit nichttoxischen Wachsen oder Gelatine vor Luft, Licht und/oder Feuchtigkeit geschützt werden.

Beispiel für die Zusammensetzung eines Kükenaufzuchtfutters, das einen erfindungsgemäßen Wirkstoff enthält:

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g Wirkstoff-Praemix ergeben nach sorgfältigem Mischen 1 kg Futter.

In einem kg Futtermischung sind enthalten:

600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg $MnSO_4 \times H_2O$, 140 mg $ZnSo_4 \times 7\ H_2O$, 100 mg $FeSO_4 \times 7\ H_2O$ und 20 mg $CuSO_4 \times 5\ H_2O$.

Der Wirkstoff-Praemix enthält die Wirkstoffe in der gewünschten Menge, z.B. 10 mg und zusätzlich 1 g DL-Methionin sowie so viel Sojabohnenmehl, daß 2,5 g Praemix entstehen.

Beispiel für die Zusammensetzung eines Schweineaufzuchtfutters, das einen erfindungsgemäßen Wirkstoff enthält:

630 g Futtergetreideschrot (zusammengesetzt aus 200 g Mais, 150 g Gerste-,. 150 g Hafer- und 130 g Weizenschrot), 80 g Fischmehl, 60 g Sojaschrot, 60 g Tapiokamehl, 38 g Beirhefe, 50 g Vitamin-Mineral-Mischung für Schweine (Zusammensetzung z.B. wie beim Kükenfutter), 30 g Leinkuchenmehl, 30 g Maiskleberfutter, 10 g Sojaöl, 10 g Zuckerrohrmelasse und 2 g Wirkstoff-Praemix (Zusammensetzung z.B. wie beim Kükenfutter) ergeben nach sorgfältigem Mischen 1 kg Futter.

Die angegebenen Futtergemische sind vorzugsweise zur Aufzucht und Mast von Küken bzw. Schweinen abgestimmt, sie können jedoch in gleicher oder ähnlicher Zusammensetzung auch zur Aufzucht und Mast anderer Tiere verwendet werden.

### Beispiel 1 (Ausgangsprodukt)

3-Chloromethyl-7β-phenylacetamido-3-cephem-4-carbonsäurebenzhydrylester

Zu einer Lösung von 103 g (0,2 Mol) 3-Hydroxymethyl-7β-phenylacetamido-3-cephem-4-carbonsäurebenzhydrylester (hergestellt z.B. nach Helv. Chim. Acta *57*, 2044 (1974)) in 3,5 l absol. Tetrahydrofuran gibt man unter Eiskühlung 24 ml (0,3 Mol) Pyridin, 400 µl Dimethylformamid und 21,6 ml (0,3 Mol) Thionylchlorid. Nach 10 Minuten wird am Rationsverdampfer eingeengt, mit 2 l Essigester aufgenommen und zweimal mit Natriumhydrogencarbonatlösung und einmal mit Wasser ausgeschüttelt. Die organische Phase wird mit je 50 g Kieselgur und Aktivkohle ausgerührt und über eine mit Kieselgel belegte Fritte abgesaugt. Anschließend wird über Magnesiumsulfat getrocknet, eingeengt, in 200 ml Methylenchlorid aufgenommen und mit Petrolether ausgefällt.

Ausbeute: 76 g

$^1$-NRM ($DCCl_3$)

δ (ppm) = 7,20—7,50 (15H, m, arom.); 6,96 (1H, s, $CHØ_2$); 6,30 (1H, d, J=9Hz, NH); 5,86 (1H, dd, J=9Hz, J=5Hz, H-7); 4,95 (1H, d, J=5Hz, H-6); 4,.36 (2H, bs, $CH_2$—Cl); 3,66 (1H, d, J=15Hz, Ø—$CH_2$—); 3,58 (1H, d, J=15Hz, Ø—$CH_2$—); 3,56 (1H, d, J=18Hz, H-2); 3,40 (1H, d, J=18hz, H-2).

### Beispiel 2 (Ausgangsprodukt)

3-(1-Methyl-1-pyrrolidinium)-methyl-7β-phenylacetamido-3-cephem-4-carboxylat

2,13 g (4 mMol) 3-Chloromethyl-7β-phenylacetamido-3-cephem-4-carbonsäurebenzhydrylester werden bei 0°C in 24 ml absol. Methylenchlorid gelöst. Nach Zugabe von 12 ml Anisol und 12 ml Trifluoressigsäure wird 25 Minuten bei 0°C gerührt. Im Vakuum wird eingeengt, 10 ml Benzol werden zugegeben und der Ansatz wird 1 h im Hockvakuum eingeengt. Der Rückstand wird in 20 ml absol. Tetrahydrofuran gelöst und 1,7 g (20 mMol) N-Methylpyrrolidin werden zugegeben. Die Lösung wird 30 Minuten bei Raumtemperatur gerührt. 100 ml Ether werden zugegeben und der Ether wird abdekantiert. Der Rückstand wird nochmals mit Ether verrührt, nach erneutem dekantieren des Ethers kurz im Vakuum getrocknet und dann in 100 ml Wasser suspendiert. Mit Ionenaustauscher MP 62 wird neutralisiert und dann über Adsorberharz HP 20 chromatographiert. (Laufmittel: Wasser/Acetonitril 95/5). Abschließend werden die Produktfraktionen lyophilisiert.

Ausbeute: 0,725 g (44%)

$^1$H-NMR (DMSO-$d_6$)

δ (ppm) = 9,21 (1H, d J=9Hz, NH); 7,25—7,35 (5H, m, arom.); 5,55 (1H, dd, J=9, J=5Hz, H-7); 5,07 (1H, d, J=5Hz, H-6); 5,00 (1H, d, J=13Hz, $CH_2$-Pyrrol.); 3,93 (1H, d, H=13Hz, $CH_2$-Pyrrol); 3,82 (1H, d, H=18Hz, S—$CH_2$); 3,60 (1H, d, J=14Hz, Ø—$CH_2$); 3,51 (1H, d, J=14Hz, Ø—$CH_2$); 3,42 (4H, m, Pyrrol.); 3,34 (1H, d, J=18Hz, S—$CH_2$); 2,92 (3H, S, $CH_3—^{\oplus}N<$—); 2,06 (4H, m, Pyrrol).

## Beispiel 3

7-Amino-3-(1-methyl-1-pyrrolidinium)methyl-3-cephem-4-carboxylat

3,32 g (8 mMol) 3-(1-Methyl-1-pyrrolidinium)methyl-7β-phenylacetamido-3-cephem-4-carboxylat werden in 100 ml Wasser gelöst. Mit 4 N Triethylamin in Ethanol wird pH 7,8 eingestellt. Anschließend werden 4 g Penicillin-G-Acylase zugegeben und der pH wird durch Zugabe von Triethylamin konstant gehalten. Nach Beendigung der enzymatischen Spaltung wird von der Acylase abfiltriert und das Filtrat mit konz. Salzsäure auf pH 2 gestellt. Vom entstandenen Niederschlag wird über Kieselgur abgesaugt und das Filtrat wird zu 2 l Aceton getropft. Das gewünschte Produkt kristallisiert als Hydrochlorid aus un wird abgesaugt und getrocknet.

Ausbeute: 1,98 g (× HCl × $H_2O$, 71%).

NMR ($D_2O$)

δ (ppm) = 5,37 (1H, d, J=5Hz, H-7); 5,16 (1H, d, J=5Hz, H-6); 4,58 (1H, d, J=14Hz, $CH_2$-Pyrrol.); 3,99 (1H, d, J=14Hz, $CH_2$-Pyrrol.); 3,93 (1H, d, J=18Hz, S—$CH_2$), 3,53 (1H, d, J=18Hz, S—$CH_2$); 3,48 (4H, m, Pyrrol.);

2,94 (3H, s, $CH_3$—$^{\oplus}$N—); 2,17 (4H, m, Pyrrol.).

## Beispiel 4

7-[1-(2-Aminothiazol-4-yl)-1(Z)-propencarboxamido]-3-(1-methyl-1-pyrrolidinium)methyl-3-cephem-4-carboxylat

659 mg (3.58 mmol) 1-(2-Aminothiazol-4-yl-1(Z)-propencarbonsäure werden unter Stickstoff bei Raumtemperatur in 4,5 ml absol. Dimethylformamid gelöst. Nach Zugabe von 230 µl N-Ethyldiisopropylamin, 250 µl Tripropylamin und 310 µl Tributylamin wird auf −50°C gekühlt. 290 µl Methansulfonsäurechlorid werden zugegeben und die Lösung wird 30 min. bei −50°C gerührt. Anschließend wird diese Lösung schnell zu einer auf 0°C gekühlten Lösung von 900 mg (2,7 mmol) 6-Amino-3-(1-methyl-1-pyrrolidinium)-methyl-3-cephem-4-carboxylat (× HCl × $H_2O$) in 1,4 ml Wasser und 1,4 ml Triethylamin gegeben. Nach 5 min. Wird die Reaktionslösung auf 400 ml Aceton gegossen. Der entstehende Niederschlag wird abgesaugt, getrocknet und über Adsorberharz HP 20 chromatographiert (Laufmittel:Wasser/Acetonitril 95/5).

Ausbeute: 530 mg (50,4%).

NMR (DMSO—$d_6$).

δ (ppm) = 9,28 (1H, d, J=9Hz, NH); 7,05 (2H, bs, $NH_2$); 6,35 (1H, q, J=8Hz, C=CH); 6,23 (1H, s, Thiazol); 5,68 (1H, dd, J=5Hz, J=9Hz, H-7-Lactam); 5,17 (1H, d, J=5Hz, H-6-Lactam); 5,01 (1H, d, J=14Hz, $CH_2$-Pyrrol); 3,93 (1H, d, J=14Hz, $CH_2$-Pyrrol.); 3,83 (1H, d, J=18Hz, S—$CH_2$); 3,45 (4H, m Pyrrol.); 3,35 (1H, d, J=18Hz, S—$CH_2$). 2,93 (3H, S, $CH_3$—N(H)—) 2,08 (4H, m, Pyrrol.); 1,79 (3H, d, J=8Hz, C=CH—$CH_3$).

## Beispiel 5

7-Amino-3-(1-methyl-1-piperidinium)methyl-3-cephem-4-carboxylat

10 g (18,8 mmol) 3-Chloromethyl-7β-phenylacetamido-3-cephem-4-carbonsäurebenzhydrylester werden bei 0°C in 112 ml absol. Methylenchlorid gelöst. Nach Zugabe von 56 ml Anisol und 56 ml Trifluoressigsäure wird 25 Minuten bei 0°C gerührt. Im Vakuum wird eingeengt, 100 ml Benzol werden zugegeben und der Ansatz wird 1 h im Hochvakuum eingeengt. Der Rückstand wird in 100 ml absol. Tetrahydrofuran gelöst und 9,3 g (94 mmol) N-Methylpiperidin werden zugegeben. Die Lösung wird 30 Minuten bei Raumtemperatur gerührt. 100 ml Ether werden zugegeben. Der entstehende Niederschlag wird abgesaugt, mit 500 ml Ether gewaschen und durch Zugabe von $NaHCO_3$ in 50 ml Wasser gelöst. Anschließend werden 4 g Penicillin-G-Acylase zugegeben und der pH-Wert wird durch Zugabe von 4N-Triethylamin in Ethanol konstant bei 7,8 gehalten. Nach Beendigung der enzymatischen Spaltung wird von der Acylase abfiltriert und das Filtrat mit konz. Salzsäure auf pH 2 gestellt. Vom entstehenden Niederschlag wird über Kieselgel abgesaugt und das Filtrat wird zu 2 l Aceton zugetropft. Das gewünsche Produkt kristallisiert als Hydrochlorid aus und wird abgesaugt und getrocknet. Ausbeute: 3,4 g (× HCl × $H_2O$, 49,5%).

NMR ($D_2O$).

δ (ppm) = 5,40 (1H, d, J=5Hz, H-7); 5,17 (1H, d, J=5Hz, H-6); 4,66 (1H, d, J=14Hz, $CH_2$-Pip); 4,03 (1H, d, J=14Hz, $CH_2$-Pip); 3,97 (1H, d, J=18Hz, S—$CH_2$); 3,54 (1H, d, J=18Hz, S—$CH_2$); 3,34 (4H, m, Pip);

2,98 (3H, s, $CH_2$—$^{\pm}$N—); 1,84 (4H, n, Pip); 1,52 (2H, m, Pip).

## Beispiel 6

7-[1-(2-Aminothiazol-4-yl)-1(Z)-propencarboxamido]-3-(1-methyl-1-piperidinium)methyl-3-cephem-4-carboxylat

Die Herstellung erfolgte analog Beispiel 4 aus 1-(2-Aminothiazol-4-yl)-1(Z)-propencarbonsäure und 7-Amino-3-(1-methyl-1-piperidinium)methyl-3-cephem-4-carboxylat.

$^1$H-NMR (DMSO-$d_6$).

δ (ppm) = 9,32 (1H, d, J=9Hz, NH); 7,07 (2H, bs, $NH_2$); 6,39 (1H 9, J=8H, C=CH); 6,27 (1H, s, Thiazol); 5,73 (1H, dd, J=5Hz, J=9Hz, H-7-Lactam); 5,21 (1H, d, J=5Hz, H-6-Lactam), 5,12 (1H, d, J=14Hz, $CH_2$-Pip.); 3,98 (1H, d, J=14Hz, $CH_2$-Pip.); 3,88 (1H, d, J=18Hz, S—$CH_2$); 3,42 (4H, m, Pip); 3,39 (1H, d, J=18Hz, S—$CH_2$); 2,98 (3H, S, $CH_3$—$^{\pm}$N—); 1,82 (7H, m, Pip, C=C—$CH_3$) 1,53 (2H, m, Pip).

## Beispiel 7

7-amino-3-chinuclidiniummethyl-3-cephem-4-carboxylat

Aus 10 g (18,2 mmol) 3-Chloromethyl-7β-phenylacetamido-3-cephem-4-carbonsäurebenzhydrylester erhält man analog Beispiel 5 2,1 g (× HCl × $H_2O$) Titelverbindung

NMR ($D_2O$).

δ (ppm) = 5,31 (1H, d, J=5Hz); 5,11 (1H, d, J=5Hz, H-6); 4,44 (1H, d, J=14Hz, $CH_2$—Chin.); 3,85 (1H, d, J=14Hz, $CH_2$-Chin.); 3,78 (1H, d, J=18Hz, S—$CH_2$); 3,42 (1H, d, J=18Hz, S—$CH_2$); 3,30 (6H, m, Chin.); 2,07 (1H, m, Chin.); 1,90 (6H, m, Chin.).

## Beispiel 8

7-[1-(2-Aminothiazol-4-yl)-1-(Z)-propencarboxamido]-3-chinuclidiniummethyl-3-cephem-4-carboxylat

Die Herstellung erfolgte analog Beispiel 4 aus 1-(2-Aminothiazol-4-yl)-1(Z)-propencarbonsäure und 7-Amino-3-chinuclidinium-methyl-3-cephem-4-carboxylat.

$^1$H-NMR (DMSO-$d_6$)

δ (ppm) = 9,28 (1H, d, J=9Hz, HN); 7,04 (2H, bs, $NH_2$); 6,35 (1H, q, J=8Hz, C=CH); 6,23 (1H, s, Thiazol); 5,70 (1H, dd, J=9Hz, J=5Hz, H-7-Lactam), 5,17 (1H, d, J=5Hz, H-6-Lactam); 4,92 (1H, d, J=14hz, $CH_2$-Chin.); 3,81 (1H, d, J=18Hz, S—$CH_2$); 3,75 (1H, d, J=14Hz, $CH_2$-Chin.); 3,38 (7H, m, S—$CH_2$-Chin.); 2,06 (1H, m, Chin.); 1,85 (6H, m, Chin); 1,80 (3H, d, J=8H, C=C—$CH_3$).

## Beispiel 9
### (Ausgangsprodukt)

3-(1-Methyl-1-pyrrolidinium)methyl-7β-phenylacetamido-3-cephem-4-carboxylat

Unter Stickstoff werden 1,56 g (4 mmol) 3-Acetoxymethyl-7β-phenylacetamido-3-cephem-4-carbonsäure bei Raumtemperatur in 16 ml absol. Methylenchlorid aufgeschlämmt und durch Zugabe von 2,56 ml (12 mmol) N-Methyl-N-trimethylsilyltrifluoracetamid (MSTFA) in Lösung gebracht. Nach Abkühlen auf 0°C werden 8 ml einer 2 molaren Lösung von Trimethylsilyljodid in Methylenchlorid zugegeben und die Reaktionslösung wird 1 Stunde bei 0°C gerührt. Nach Zugabe von 2,52 ml (30,8 mmol) absol. Tetrahydrofuran wird weitere 15 Minuten bei 0°C gerührt. Anschließend werden 3,4 g (40 mmol) N-Methylpyrrolidin zugegeben und die Lösung wird 30 Minuten nachgerührt. Dann werden 0,8 ml Wasser und nach weiteren 5 Minuten 100 ml absol. Ether zugegeben. Der Ether wird abdekantiert, der Rückstand nochmals mit Ether verrührt und nach erneutem Dekantieren mit im Vakuum getrocknet. Abschließend wird 100 ml Wasser aufgenommen und über Adsorberharz HP 20 chromatographiert (Laufmittel:Wasser/Acetonitril 95/5).

Nach Gefriertrocknung der Produktfraktionen erhält man 1,31 g (79%) Produkt, welches mit dem in Beispiel 2 hergestellten Produkt identisch ist.

## Beispiel 10

7-[Z-2-(2-Aminothiazol-4-yl)-2-benzylidenacetamido]-3-(1-methyl-1-pyrrolidinium)methyl-3-cephem-4-carboxylat

Eine Lösung von 0,7 g Z-2-(2-Aminothiazol-4-yl)-2-benzylidenessigsäure in 10 ml Dimethylformamid wird mit 0,4 g 1-hydroxybenztriazol und 0,6 g N,N'-Dicyclohexylcarbodiimid versetzt und vier Stunden bei Raumtemperatur gerührt. Man saugt vom ausgefallenen Harnstoff ab und versetzt die Mutterlauge mit eine Lösung von 0,8 g 7-Amino-3-(1-methyl-1-pyrrolidinium)methyl-3-cephem-4-carboxylat (× HCl × $H_2O$) und 1,3 ml Triethylamin in 1,3 ml Wasser. Nach vierstündigem Rühren bei Raumtemperatur wird die Reaktionslösung in 500 ml Aceton eingerührt und der ausgefallene Niederschlag abgesaugt und getrocknet.

Ausbeute: 0,43 g.

NMR ($D_2O$):

δ (ppm) = 7,45 (5H, bs), 7,37 (1H, S), 6,70 (1H, s), 5,87 (1 H, d, J=8Hz), 5,35 (1H, J=14Hz), 5,33 (1H, J=5Hz), 4,05 (1H, J=14Hz), 2,85 (1H, J=18Hz), 4,03 (5H, m), 2,97 (3 H, s), 2,22 (4H, m).

## Beispiel 11

7-Amino-3-(4-methyl-4-morpholinium)methyl-3-cephem-4-carboxylat

Die Herstellung erfolgt analog Beispiel 5 aus 3-Chloromethyl-7β-phenylacetamido-3-cephem-4-carbonsäurebenzhydrylester.

$^1$H-NMR ($D_2O$).

δ (ppm) = 5.29 (1H, d, J=5Hz, H-7-Lactam); 5.07 (1H, d, J=5Hz, H-6-Lactam); 4,74 (1H, d, J=14Hz, $CH_2$-Morph.); 4.04 (1H, d, J=14Hz, $CH_2$-Morph.); 3.92 (4H, m, Morph.); 3.85 (1H, d, J=18Hz, S—$CH_2$); 3.46 (1H, d, J=18Hz, S—$CH_2$); 3.35 (4H, m, Morph.); 3.05 (3H, s, $CH_3{-}^{\pm}\overset{|}{\underset{|}{N}}{-}$).

Beispiel 12

7-[1-(2-Aminothiazol-4-yl)-1(Z)-propencarboxamido]-3-(4-methyl-4-morpholinium)methyl-3-cephem-4-carboxylat

Die Herstellung erfolgt analog Beispiel 4 aus 1-(2-Aminothiazol-4-yl)-1(Z)-propencarbonsäure und 7-Amino-3-(4-methyl-4-morpholinium)methyl-3-cephem-4-carboxylat.

$^1$H-NMR (DMSO-$D_6$)

δ (ppm) = 9.28 (1H, d, J=9Hz, NH); 7.03 (2H, bs, $NH_2$); 6.35 (1H, q, J=8Hz, C=C—H); 6.23 (1H, s, Thiazol); 5.69 (1H, dd, J=9Hz, J=5Hz, H-7-Lactam; ; 5.18 (2H, m, H-6-Lactam, $CH_2$-Morph.); 3.80—4.10 (6H, m, $CH_2$-Morph., S—$CH_2$, Morph.); 3.30—3.50 (5H, m, S—$CH_2$, Morph.); 3.07 (3H, S, $CH_3{-}^{\pm}\overset{|}{\underset{|}{N}}{-}$); 1.79 (3H, d, J=8Hz, C=C—$CH_3$).

Beispiel 13

7-Amino-3-(trimethylammonium)methyl-3-cephem-4-carboxylat

Die Herstellung erfolgt analog Beispiel 5 aus 3-Chloromethyl-7β-phenylacetamido-3-cephem-4-carbonsäurebenzhydrylester.

$^1$H-NMR ($D_2O$).

δ (ppm) = 5.34 (1H, d, J=5Hz, H-7-Lactam); 5.12 (1H, d, J=5Hz, H-6-Lactam); 4.61 (1H, d, J=13Hz, $CH_2$-Ammon.);3.97 (1H, d, J=13Hz, $CH_2$-Ammon.); 3.92 (1H, d, J=18Hz, S—$CH_2$); 3.47 (1H, d, J=18Hz, S—$CH_2$); 3.03 (9H, s, $CH_3{-}^{\pm}\overset{|}{\underset{|}{N}}{-}$).

Beispiel 14

7-[1-(2-Aminothiazol-4-yl)-1(Z)-propencarboxamido]-3-(trimethylammonium)methyl-3-cephem-4-carboxylat

Die Herstellung erfolgt analog Beispiel 4 aus 1-(2-Aminothiazol-4-yl)-1(Z)-propencarbonsäure und 7-amino-3-(trimethylammonium) methyl-3-cephem-4-carboxylat.

$^1$H-NMR (DMSO-$d_6$).

δ (ppm) = 9.27 (1H, d, J=9Hz, NH); 7.02 (2H, bs, $NH_2$); 6.33 (1H, q, J=8Hz, C=C—H); 6.22 (1H, s, Thiazol); 5.68 (1H, dd, J=9Hz, J=5Hz, H-7-Lactam); 5.19 (1H, d, J=5Hz, H-6-Lactam); 5.00 (1H, d, J=13Hz, —$CH_2$-Ammon.); 3.91 (1H, d, J=13Hz, $CH_2$-Ammon.); 3.85 (1H, d, J=18Hz, S—$CH_2$); 3.31 (1H, d, J=18Hz, S—$CH_2$); 3.00 (9H, s, ${-}^{\pm}\overset{|}{\underset{|}{N}}{-}$); 1.79 (3H, d, J=8Hz, C=C—$CH_3$).

Beispiel 15

7-Amino-3-(dimethylethylammonium)methyl-3-cephem-4-carboxylat

Die Herstellung erfolgt analog Beispiel 5 aus 3-Chloromethyl-7β-phenylacetamido-3-cephem-4-carbonsäurebenzhydrylester.

$^1$H-NMR ($D_2O$).

δ (ppm) = 5.31 (1H, d, J=5Hz, H-7-Lactam); 5.10 (1H, d, J=5Hz, H-6-Lactam); 4.57 (1H, d, J=13Hz, $CH_2$-Ammon.); 3.89 (1H, d, J=13Hz, $CH_2$-Ammon.); 3.87 (1H, d, J=18Hz, S—$CH_2$); 3.46 (1H, d, J=18Hz, S—$CH_2$); 3.29 (2H, m, $CH_2{-}^{\pm}\overset{|}{\underset{|}{N}}{-}$); 2.93 (3H, s, $CH_3{-}^{\pm}\overset{|}{\underset{|}{N}}{-}$); 2.88 (3H, s, $CH_3{-}\overset{H}{\underset{|}{N}}{-}$); 1.25 (3H, t, J=7Hz, $CH_3$).

Beispiel 16

7-[1-(2-Aminothiazol-4-yl)-1(Z)-propencarboxamido]-3-(dimethylethylammonium)methyl-3-cephem-4-carboxylat

Die Herstellung erfolgt analog Beispiel 4 aus 1-(2-Aminothiazol-4-yl)-1(Z)-propencarbonsäure und 7-Amino-3-(dimethylethylammonium)methyl-3-cephem-4-carboxylat.

$^1$H-NMR (DMSO-$d_6$).

δ (ppm) = 9.27 (1H, d, J=9Hz, NH); 7.03 (2H, bs, $NH_2$); 6.33 (1H, q, J=8Hz, C=C—H); 6.22 (1H, s, Thiazol); 5.68 (1H, dd, J=9hz, J=5Hz, H-7-Lactam); 5.18 (1H, d, J=5Hz, H-6-Lactam); 5.05 (1H, d, J=13Hz,

$CH_2$-Ammon.); 3.96 (1H, d, J=13Hz, $CH_2$-Ammon.); 3.94 (1H, d, J=18Hz, S—$CH_2$); 3.36 (1H, m, $CH_2$—$\overset{+}{-}$N—, S—$CH_2$); 2.94 (3H, s, $CH_3$—$\overset{+}{-}$N—); 2.88 (3H, s, $CH_3$—$\overset{+}{-}$N—); 1.78 (3H, d, J=8Hz, C=C—$CH_3$); 1.25 (3H, t, J=7Hz, $CH_3$).

Beispiel 17

7-amino-3-(1-ethyl-1-pyrrolidinium)methyl-3-cephem-4-carboxylat

10 g (18.8 mmol) 3-Chloromethyl-7β-phenylacetamido-3-cephem-4-carbonsäurebenzhydrylester werden bei 0°C in 112 ml absol. Methylenchlorid gelöst. Nach Zugabe von 56 ml Anisol und 56 ml Trifluoressigsäure wird 25 Minuten bei 0°C gerührt. Im Vakuum wird eingeengt, 100 ml Benzol werden zugegeben und der Ansatz wird 1 h im Hochvakuum eingeengt. Der Rückstand wird in 10 ml absol. Tetrahydrofuran gelöst und 18.6 g (188 mmol) N-Ethylpyrrolidin werden zugegeben. Die Lösung wird 30 Minuten bei Raumtemperatur gerührt. 100 ml Ether werden zugegeben. Der entstehende Niederschlag wird abgesaugt, mit 500 ml Ether gewaschen und durch Zugabe von $NaHCO_3$ in 50 ml Wasser gelöst. Anschließend werden 4 g immobilisierter Penicillin-G-Acylase zugegeben und der pH-Wert wird durch Zugabe von 4N-Triethylamin in Ethanol konstant bei 7.8 gehalten. Nach Beendigung der enzymatischen Spaltung wird von der Acylase abfiltriert und das Filtrat mit konz. Salzsäure auf pH 2 gestellt. Vom entstehenden Niederschlag wird über Kieselgel abgesaugt und das Filtrat wird zu 2 l Aceton getropft. Das gewünschte Produkt kristallisiert als Hydrochlorid aus und wird abgesaugt und getrocknet.

Ausbeute: 1.76 g (× HCl × $H_2O$, 25,6%).

NMR ($D_2O$).

δ (ppm) = 5.31 (1H, d, J=5Hz, H-7-Lactam); 5.12 (1H, d, J=5Hz, H-6-Lactam); 4.62 (1H, d, J=14Hz, $CH_2$-Pyrrol.); 3.88 (1H, d, J=14Hz, $CH_2$-Pyrrol.); 3.86 (1H, d, J=18Hz, S—$CH_2$); 3.58 (1H, d, J=18hz, S—$CH_2$); 3.42 (4H, m, Pyrrol.); 3.24 (2H, q, J=7Hz, —$CH_2$—$\overset{+}{-}$N—); 2.06 (4H, m, Pyrrol.); 1.24 (3H, t, J=7Hz, $CH_3$).

Beispiel 18

7-[1-(2-Aminothiazol-4-yl)-1(Z)-propencarboxamido]-3-(1-ethyl-1-pyrrolidinium)methyl-3-cephem-4-carboxylat

Die Herstellung erfolgt analog Beispiel 4 aus 1-(2-Aminothiazol-4-yl)-1(Z)-propencarbonsäure und 7-Amino-3-(1-ethyl-1-pyrrolidinium)methyl-3-cephem-4-carboxylat.

$^1$H-NMR (DMSO-$d_6$).

δ (ppm) = 9.27 (1H, d, J=9Hz, NH); 7.02 (2H, bs, $NH_2$); 6.33 (1H, q, J=8Hz, C=C—H); 6.22 (1H, s, Thiazol); 5.67 (1H, dd, J=9Hz, J=5Hz, H-7-Lactam); 5.15 (1H, d, J=5Hz, H-6-Lactam); 5.05 (1H, d, J=14Hz, $CH_2$-Pyrrol.); 3.83 (1H, d, J=14Hz, $CH_2$-Pyrrol.); 3.79 (1H, d, J=18Hz, S—$CH_2$); 3.30—3.50 (7H, m); 2.00 (4H, m, Pyrrol.); 1.79 (3H, d, J=8Hz, C=C—$CH_3$); 1.26 (3H, t, J=7Hz, $CH_3$).

Beispiel 19

(Ausgangsprodukt)

3-[1-(2-Hydroxyethyl)-1-pyrrolidinium]methyl-7β-phenylacetamido-3-cephem-4-carboxylat

Unter Stickstoff werden 4.68 g (12 mmol) 3-Acetoxymethyl-7β-phenylacetamido-3-cephem-4-carbonsäure bei Raumtemperatur in 48 ml absol. Methylenchlorid aufgeschlämmt und durch Zugabe von 7.6 ml (36 mmol) N-Methyl-N-Trimethylsilyltrifluoracetamid (MSTFA) in Lösung gebracht. Nach Abkühlen auf 0°C werden 7 ml (48 mmol) Trimethylsilyljodid zugegeben und die Reaktionslösung wird 1 h bei 0°C gerührt. Anschließend werden 14.4 ml (120 mmol) N-(2-Hydroxyethyl)pyrrolidin zugegeben und die Lösung wird 30 Minuten nachgerührt. Dann werden 2.4 ml Wasser zugegeben und nach weiteren 5 Minuten wird auf 200 ml Ether gegossen. Der Ether wird vom öligen Rückstand abdekantiert, der Rückstand nochmals mit Ether verrührt und nach erneutem Dekantieren in Wasser aufgenommen und über Adsorberharz HP 20 chromatographiert (Elutionsmittel:Acetonitril/Wasser 5/95).

Ausbeute: 3.6 g (68%).

$^1$H-NMR ($D_6$-DMSO).

δ (ppm) = 9.13 (1H, d, J=9Hz, NH); 7.28 (5H, m, arom.); 5.55 (1H, dd, J=9Hz, J=5Hz, H-7-Lactam); 5.06 (1H, d, J=5Hz, H-6-Lactam); 5.04 (1H, d, J=14Hz, $CH_2$-Pyrrol.); 3.95 (1H, d, J=14Hz, $CH_2$-Pyrrol.); 3.33—3.85 (12H, m); 2.04 (4H, m, Pyrrol.).

Beispiel 20

7-Amino-3-[1-(2-hydroxyethyl)-1-pyrrolidinium]methyl-3-cephem-4-carboxylat

Zu einer Lösung von 3.5 g (7.8 mmol) 3-[1-(2-Hydroxyethyl-1-pyrrolidinium]methyl-7β-phenylacetamido-3-cephem-4-carboxylat in 100 ml Wasser werden 4 g immobilisierter Penicillin-G-Acylase gegeben und der pH-Wert wird durch Zugabe von 4N Triethylamin in Ethanol konstant bei 7.8 gehalten.

Nach Beendigung der enzymatischen Spaltung wird von der Acylase abfiltriert und das Filtrat wird mit konz. Salzsäure auf pH 2 gestellt. Vom entstehenden Niederschlag wird über Kieselgel abgesaugt und das Filtrat wird zu 2 l Aceton getropft. Das gewünschte Produkt kristallisiert als Hydrochlorid aus und wird abgesaugt und getrocknet.

Ausbeute: 1.9 g (× HCl × $H_2O$, 64%).

$^1$H-NMR ($D_6$-DMSO).

(ppm) = 5.33 (1H, d, J=5Hz, H-7-Lactam); 5.13 (1H, d, J=5Hz, H-6-Lactam); 4.70 (1H, d, J=14Hz, $CH_2$-Pyrrol); 3.93 (2H, m, $CH_2$—OH); 3.87 (1H, d, J=18Hz, S—$CH_2$); 3.30—370 (7H, m); 2.11 (4H, m, Pyrrol.).

Beispiel 21

7-[1-(2-Aminothiazol-4-yl)-1(Z)-propencarboxamido]-3-[1-(2-hydroxyethyl)-1-pyrrolidinium]methyl-3-cephem-4-carboxylat

Die Herstellung erfolgt analog Beispiel 4 aus 1-(2-Aminothiazol-4-yl)-1(Z)-propencarbonsäure und 7-Amino-3-[1-(2-hydroxyethyl)-1-pyrrolidinium]methyl-3-cephem-4-carboxylat.

$^1$H-NMR (DMSO-$d_6$).

(ppm) = 9.25 (1H, d, J=9Hz); 7.00 (2H, bs, $NH_2$); 6.31 (1H, q, J=8Hz, C=C—H); 6.19 (1H, s, Thiazol); 5.65 (1H, dd, J=9Hz, J=5Hz, H-7-Lactam); 5.14 (1H, d, J=5Hz, H-6-Lactam); 5.04 (1H, d, J=13Hz, $CH_2$-Pyrrol.); 3.80 (2H, m, $CH_2$—OH); 3.77 (1H, d, J=18Hz, S—$CH_2$); 3.30—3.60 (7H, m); 2.01 (4H, m, Pyrrol.); 1.76 (3H, d, J=8Hz, C=C—$CH_3$).

Beispiel 22

(Ausgangsprodukt)

3-[1-(2-Hydroxyethyl)-1-piperidinium]methyl-7β-phenylacetamido-3-cephem-4-carboxylat

Die Herstellung erfolgt analog Beispiel 19 aus 3-Acetoxymethyl-7β-phenylacetamido-3-cephem-4-carbonsäure und N-(2-Hydroxyethyl)-piperidin.

$^1$H-NMR ($D_6$-DMSO).

δ (ppm) = 9.17 (1H, d, J=9Hz, NH); 7.30 (5H, m.arom.); 5.56 (1H, dd, J=9Hz, J=5Hz, H-7-Lactam); 5.09 (1H, d, J=5Hz, H-6-Lactam); 5.08 (1H, d, J=13Hz, $CH_2$-Pip.); 3.10—3.90 (12H, m); 1.40—1.90 (6H, m).

Beispiel 23

7-Amino-3-[1-(2-hydroxyethyl)-1-piperidinium]methyl-3-cephem-4-carboxylat

Die Herstellung erfolgt analog Beispiel 20 aus 3-[1-(2-Hydroxyethyl)-1-piperidinium]methyl-7β-phenylacetamido-3-cephem-4-carboxylat.

$^1$H-NMR ($D_2O$)

δ (ppm) = 5.34 (1H, d, J=5Hz, H-7-Lactam); 5.14 (1H, d, J=5Hz, H-6-Lactam); 4.75 (1H, d, J=14Hz, $CH_2$-Pip.); 3.96 (2H, m, $CH_2$—OH); 3.91 (1H, d, J=18Hz, S—$CH_2$); 3.10—3.60 (7H, m); 1.40—1.90 (6H, m Pip.).

Beispiel 24

7-[1-(2-Aminothiazol-4-yl)-1(Z)-propencarboxamido]-3-[1-(2-hydroxyethyl)-1-piperidinium]methyl-3-cephem-4-carboxylat

Die Herstellung erfolgt analog Beispiel 4 aus 1-(2-Aminothiazol-4-yl)-1(Z)-propencarbonsäure und 7-Amino-3-[1-(2-hydroxyethyl)-1-piperidinium]methyl-3-cephem-4-carboxylat.

$^1$H-NMR (DMSO-$d_6$).

δ (ppm) = 9.27 (1H, d, J=9Hz, NH); 7.02 (2H, bs, $NH_2$); 6.33 (1H, q, J=8Hz, C=C—H); 5.69 (1H, dd, J=9Hz, J=5Hz, H-7-Lactam); 5.17 (1H, d, J=5Hz, H-6-Lactam); 5.09 (1H, d, J=13Hz, $CH_2$-Pip.); 4.00 (1H, d, J=13Hz, $CH_2$-Pip.); 3.82 (3H, m); 3.10—3.50 (7H, m); 1.40—1.90 (6H, m, Pip.); 1.78 (3H, d, J=8Hz, C=C—$CH_3$).

Beispiel 25

(Ausgangsprodukt)

3-[4-(2-Hydroxyethyl)-4-morpholinium]methyl-7β-phenylacetamido-3-cephem-4-carboxylat

Die Herstellung erfolgt analog Beispiel 19 aus 3-Acetoxymethyl-7β-phenylacetamido-3-cephem-4-carbonsäure und N-(2-Hydroxyethyl)morpholin

$^1$H-NMR (DMSO-$d_6$).

δ (ppm) = 9.19 (1H, d, J=9Hz, NH); 7.34 (5H, m, arom.); 5.62 (1H, dd, J=9Hz, J=5Hz, H-7-Lactam); 5.20 (1H, d, J=14Hz, $CH_2$-Morph.); 5.13 (1H, d, J=5Hz, H-6-Lactam); 4.16 (1H, d, J=14Hz, $CH_2$-Morph.); 3.30—4.10 (16H, m).

Beispiel 26

7-Amino-3-[4-(2-hydroxyethyl)-4-morpholinium]methyl-3-cephem-4-carboxylat

Die Herstellung erfolgt analog Beispiel 20 aus 3-[4-(2-Hydroxyethyl)-4-morpholinium]methyl-7β-phenylacetamido-3-cephem-4-carboxylat.

$^1$H-NMR ($D_2O$)

δ (ppm) = 5.36 (1H, d, J=5Hz, H-7-Lactam); 5.15 (1H, d, J=5Hz, H-6-Lactam); 4.88 (1H, d, J=14Hz, $CH_2$-Morph.); 4.21 (1H, d, J=14Hz, $CH_2$-Morph.); 3.30—4.10 (14H, m).

Beispiel 27

7-[1-(2-Aminothiazol-4-yl)-1(Z)-propencarboxamido]-3-[4-(2-hydroxyethyl)-4-morpholinium]methyl-3-cephem-4-carboxylat

Die Herstellung erfolgt analog Beispiel 4 aus 1-(2-Aminothiazol-4-yl)-1(Z)-propencarbonsäure und 7-Amino-3-[4-(2-hydroxyethyl)-4-morpholinium]methyl-3-cephem-4-carboxylat.

$^1$H-NMR (DMSO-$d_6$).

(ppm) = 9,31 (1H, d, J=9Hz, NH); 7,05 (2H, bs, $NH_2$); 6,37 (1H, q, J=8Hz, C=C—H); 6,25 (1H, s, Thiazol); 5.73 (1H, d, J=9Hz, J=5Hz, H-7-Lactam); 5,21 (1H, d, J=5Hz, H-6-Lactam); 5,19 (1H, d, J=14Hz, $CH_2$-Morph.); 4,14 (1H, d, J=14Hz, $CH_2$-Morph.); 3,30—4,10 (14H, m); 1,81 (3H, d, J=8Hz, C=C—H).

Beispiel 28

7-Amino-3-(1-ethyl-1-piperidinium)methyl-3-cephem-4-carboxylat

Die Herstellung erfolgt analog Beispiel 17 aus 3-Chloromethyl-7β-phenylacetamido-3-cephem-4-carbonsäurebenzhydrylester und N-Ethylpiperidin.

$^1$H-NMR ($D_2O$).

δ (ppm) = 5,31 (1H, d, J=5Hz, H-7-Lactam); 5,10 (1H, d, J=5Hz, H-6-Lactam); 4,59 (1H, d, J=15Hz, $CH_2$-Pip.); 3.89 (1H, d, J=18Hz, S—$CH_2$); 3,87 (1H, d, J=15Hz, $CH_2$-Pip.); 3,45 (1H, d, J=18Hz, S—$CH_2$);

3,34 (2H, q, J=7Hz, —$CH_2$—$\overset{\pm}{N}$—);

3,00—3,20 (4H, m, Pip.); 1,40—1,70 (6H, m, Pip.); 1,19 (3H, t, J=7Hz, $CH_3$).

Beispiel 29

7-[1-(2-Aminothiazol-4-yl)-1(Z)-propencarboxamido]-3-(1-ethyl-1-piperidinium)methyl-3-cephem-4-carboxylat

Die Herstellung erfolgt analog Beispiel 4 aus 1-(2-Aminothiazol-4-yl)-1(Z)-propencarbonsäure und 7-Amino-3-(1-ethyl-1-piperidinium)methyl-3-cephem-4-carboxylat

$^1$H-NMR (DMSO-$d_6$).

δ (ppm) = 9,28 (1H, d, J=9Hz, NH); 7,03 (2H, bs, $NH_2$); 6,35 (1H, q, J=8Hz, C=CH); 6,23 (1H, s, Thiazol); 5,69 (1H, dd, J=9Hz, J=5Hz, H-7-Lactam); 5,19 (1H, d, J=5Hz, H-6-Lactam); 5,15 (1H, d, J=15Hz, $CH_2$-Pip.); 3,85 (2H, m, $CH_2$-Pip., S—$CH_2$); 3,38 (5H, m, S—$CH_2$, Pip.); 3,20 (2H, —$CH_2$—$\overset{\pm}{N}$—); 1,81 (3H, t, J=8Hz, C=C—$CH_3$); 1,40—1,80 (6H, m, Pip.); 1,22 (3H, t, J=7Hz, $CH_3$).

Beispiel 30

7-Amino-3-(4-ethyl-4-morpholinium)methyl-3-cephem-4-carboxylat

Die Herstellung erfolgt analog Beispiel 17 aus 3-Chloromethyl-7β-phenylacetamido-3-cephem-4-carbonsäurebenzhydrylester.

$^1$H-NMR (DMSO-$d_6$).

δ (ppm) = 5,35 (1H, d, J=5Hz, H-7-Lactam); 5,16 (1H, d, J=5Hz, H-6-Lactam); 4,58 (1H, d, J=14Hz, $CH_2$-Morph.); 4,02 (1H, d, J=14Hz, $CH_2$-Morph.); 3,96 (4H, m, Morph.); 3,90 (1H, d, J=18Hz, S—$CH_2$);

3,30—3,60 (7H, m, S—$CH_2$, Morph., —$CH_2$—$\overset{\pm}{N}$—); 1,24 (3H, t, J=7Hz, $CH_3$).

Beispiel 31

7-[1-(2-Aminothiazol-4-yl)-1(Z)-propencarboxamido]-3-(4-ethyl-4-morpholinium)methyl-3-cephem-4-carboxylat

Die Herstellung erfolgt analog Beispiel 4 aus 1-(2-Aminothiazol-4-yl)-1(Z)-propencarbonsäure und 7-Amino-3-(4-ethyl-4-morpholinium)methyl-3-cephem-4-carboxylat.

$^1$H-NMR (DMSO-$d_6$).

δ (ppm) = 9,26 (1H, d, J=9Hz, NH); 7,01 (2H, bs, $NH_2$); 6,33 (1H, q, J=8Hz, (C=C—H); 6,21 (1H, s, Thiazol); 5,69 (1H, dd, J=9Hz, J=5Hz, H-7-Lactam); 5,17 (2H, m, H-6-Lactam, $CH_2$-Morph.); 3,75—4,00 (6H, m, $CH_2$-Morph., Morph., S—$CH_2$; 3,10—3,70 (7H, Morph., —$CH_2$—$\overset{\pm}{N}$—, S—$CH_2$); 1,78 (3H, d, J=8Hz, C=C—$CH_3$); 1,23 (3H, t, J=7Hz, $CH_3$).

### Beispiel 32

7-Amino-3-(1-propyl-1-pyrrolidinium)methyl-3-cephem-4-carboxylat

Die Herstellung erfolgt analog Beispiel 17 aus 3-Chloromethyl-7β-phenylacetamido-3-cephem-4-carbonsäurebenzhydrylester und N-Propylpyrrolidin.

$^1$H-NMR ($D_2O$).

δ (ppm) = 5,33 (1H, d, J=5Hz, H-7-Lactam); 5,15 (1H, d, J=5Hz, H-6-Lactam); 4,63 (1H, d, J=13Hz, $CH_2$-Pyrrol.); 3,96 (1H, d, J=13Hz, $CH_2$-Pyrrol.); 3,88 (1H, d, J=18Hz, S—$CH_2$); 3,55 (1H, d, J=18Hz, S—$CH_2$); 3,45 (4H, m, Pyrrol.); 3,12 (2H, m, $—CH_2—\overset{|}{\underset{|}{\overset{\pm}{N}}}—$); 2,10 (4H, m, Pyrrol.); 1,66 (2H, m, —$CH_2$); 0,85 (3H, m, $CH_3$).

### Beispiel 33

7-[1-(2-Aminothiazol-4-yl)-1(Z)-propencarboxamido]-3-(1-propyl-1-pyrrolidinium)methyl-3-cephem-4-carboxylat

Die Herstellung effolgt analog Beispiel 4 aus 1-(2-Aminothiazol-4-yl)-1(Z)-propencarbonsäure und 7-Amino-3-(1-propyl-1-pyrrolidinium)methyl-3-cephem-4-carboxylat.

$^1$H-NMR

δ (ppm) = 9,25 (1H, d, J=9Hz, NH); 7,02 (2H, bs, $NH_2$); 6,34 (1H, q, J=8Hz, C=CH); 6,23 (1H, s, Thiazol), 5,68 (1H, dd, J=9Hz, J=5Hz, H-7-Lactam); 5,17 (1H, d, J=5Hz, H-6-Lactam); 5,08 (1H, d, J=13Hz, $CH_2$-Pyrrol.); 3,86 (1H, d, J=13Hz, $CH_2$— Pyrrol.); 3,81 (1H, d, J=18Hz, S—$CH_2$); 3,40 (5H, m, Pyrrol., S—$CH_2$);

3,12 (2H, m, $—CH_2—\overset{|}{\underset{|}{\overset{\pm}{N}}}—$);

2,07 (4H, m, Pyrrol.); 1,80 (5H, m, —$CH_2$—, C=C—$CH_3$); 0,91 (3H, t, J=7Hz, $CH_3$).

### Beispiel 34

7-Amino-3-(1-isopropyl-1-pyrrolidinium)methyl-3-cephem-4-carboxylat

Die Herstellung erfolgt analog Beispiel 17 aus 3-Chloromethyl-7β-phenylacetamido-3-cephem-4-carbonsäurebenzhydrylester und N-Isopropylpyrrolidin.

$^1$H-NMR ($D_2O$).

δ (ppm) = 5,35 (1H, d, J=5Hz, H-7-Lactam); 5,13 (1H, d, J=5Hz, H-6-Lactam); 4,62 (1H, d, J=13Hz, $CH_2$-Pyrrol.); 4,02 (1H, d, J=13Hz, $CH_2$-Pyrrol.); 3,93 (1H, d, J=18Hz, S—$CH_2$);

3,40—3,80 (6H, m, S—$CH_2$, Pyrrol., $CH—\overset{|}{\underset{|}{\overset{\pm}{N}}}—$); 2,10 (4H, m, Pyrrol.); 1,43 (6H, m, Isoprop.).

### Beispiel 35

7-[1-(2-Aminothiazol-4-yl)-1(Z)-propencarboxamido]-3-(1-isopropyl-1-pyrrolidinium)methyl-3-cephem-4-carboxylat

Die Herstellung erfolgt analog Beispiel 4 aus 1-(2-Aminothiazol-4-yl)-1(Z)-propencarbonsäure und 7-Amino-3-(1-isopropyl-1-pyrrolidinium)methyl-3-cephem-4-carboxylat.

$^1$H-NMR (DMSO-$d_6$).

δ (ppm) = 9,28 (1H, d, J=9Hz, NH); 7,04 (2H, bs, $NH_2$); 6,37 (1H, q, J=8Hz, C=CH); 6,25 (1H, s, Thiazol); 5,70 (1H, dd, J=9Hz, J=5Hz, H-7-Lactam); 5,20 (1H, d, J=5Hz, H-6-Lactam); 4,95 (1H, d, J=13Hz, $CH_2$-Pyrrol.); 3,93 (1H, d, J=13Hz, $CH_2$-Pyrrol.); 3,77 (1H, d, J=18Hz, S—$CH_2$);

3,40—3,70 (6H, m, $CH—\overset{|}{\underset{|}{\overset{\pm}{N}}}—$, S—$CH_2$ S—$CH_2$, Pyrrol.);

1,99 (4H, m, Pyrrol.); 1,82 (3H, d, J=8Hz, C=C—$CH_3$); 1,35 (6H, m, Isoprop.).

### Beispiel 36

7-Amino-3-(1-butyl-1-pyrrolidinium)methyl-3-cephem-4-carboxylat

Die Herstellung erfolgt analog Beispiel 17 aus 3-Chloromethyl-7β-phenylacetamido-3-cephem-4-carbonsäurebenzhydrylester und N-Butyl-pyrrolidin.

$^1$H-NMR ($D_2O$).

δ (ppm) = 5,24 (1H, d, J=5Hz, H-7-Lactam); 5,04 (1H, d, J=5Hz, H-6-Lactam); 4,58 (1H, d, J=13Hz, $CH_2$-Pyrrol.); 3,85 (1H, d, J=13Hz, $CH_2$-Pyrrol.); 3,82 (1H, d, J=18Hz, S—$CH_2$); 3,43 (1H, d, J=18Hz, S—$CH_2$); 3,28 (4H, m, Pyrrol.);

3,07 (2H, m, $-CH_2-\overset{|}{\underset{|}{\overset{+}{N}}}-$);

2,04 (4H, m, Pyrrol.); 1,58 (2H, m, $-CH_2-$); 1,18 (2H, m, $-CH_2-$); 0,80 (3H, t, J=7Hz, $CH_3$).

Beispiel 37

7-[1-(2-Aminothiazol-4-yl)-1(Z)-propencarboxamido]-3-(1-butyl-1-pyrrolidinium)methyl-3-cephem-4-carboxylat

Die Herstellung erfolgt analog Beispiel 4 aus 1-(2-Aminothiazol-4-yl)-1(Z)-propencarbonsäure und 7-Amino-3-(1-butyl-1-pyrrolidinium)methyl-3-cephem-4-carboxylat.

$^1$H-NMR (DMSO-$d_6$).

δ (ppm) = 9,24 (1H, d, J=9Hz, NH); 6,98 (2H, bs, $NH_2$); 6,24 (1H, q, J=8Hz, C=CH); 6,18 (1H, s, Thiazol); 5,62 (1H, dd, J=9Hz, J=5Hz, H-7-Lactam); 5,12 (1H, d, J=5Hz, H-6-Lactam); 5,01 (1H, d, J=13Hz, $CH_2$-Pyrrol.); 3,82 (1H, d, J=13Hz, $CH_2$-Pyrrol.); 3,76 (1H, d, J=18Hz, S-$CH_2$); 3,30 (5H, m, S—$CH_2$, Pyrrol);

3,13 (2H, m, $-CH_2-\overset{|}{\underset{|}{\overset{+}{N}}}-$);

2,00 (4H, m, Pyrrol.); 1,77 (3H, d, J=8Hz, C=C—$CH_3$); 1,68 (2H, m, $-CH_2-$); 1,28 (2H, m, $-CH_2-$); 0,90 (3H, t, J=7Hz, $CH_3$).

Beispiel 38

(Ausgangsprodukt)

3-[1-(3-Hydroxypropyl)-1-pyrrolidinium]methyl-7β-phenylacetamido-3-cephem-4-carboxylat

Die Herstellung erfolgt analog Beispiel 19 aus 3-Acetoxymethyl-7β-phenylacetamido-3-cephem-4-carbonsäure und N-(3-Hydroxypropyl)-pyrrolidin.

$^1$H-NMR ($D_6$-DMSO).

δ (ppm) = 9,16 (1H, d, J=9Hz, NH); 7,30 (5H, arom.); 5,58 (1H, dd, J=9Hz, J=5Hz, H-7-Lactam); 5,08 (1H, d, J=5Hz, H-6-Lactam); 5,03 (1H, d, J=13Hz, $CH_2$-Pyrrol.); 3,88 (1H, d, J=13Hz, $CH_2$-Pyrrol.); 3,84 (1H, d, J=18Hz, S—$CH_2$); 2,90 —3,60 (11H, m); 1,80—2,10 (6H, m).

Beispiel 39

7-Amino-3-[1-(3-hydroxypropyl)-1-pyrrolidinium]methyl-3-cephem-4-carboxylat

Die Herstellung erfolgt analog Beispiel 20 aus 3[1-(3-Hydroxypropyl)-1-pyrrolidinium]methyl-3-cephem-4-carboxylat.

$^1$H-NMR ($D_2O$)

δ (ppm) = 5,31 (1H, d, J=5Hz, H-7-Lactam); 5,11 (1H, d, J=5Hz, H-6-Lactam); 4,63 (1H, d, J=13Hz, $CH_2$-Pyrrol.); 3,96 (1H, d, J=13Hz, $CH_2$-Pyrrol.); 3,88 (1H, d, J=18Hz, S—$CH_2$); 3,40—3,60 (9H, m); 2,08 (4H, m, Pyrrol.); 1,92 (2H, m, $-CH_2$).

Beispiel 40

7-[1-(2-Aminothiazol-4-yl)-1(Z)-propencarboxamido]-3-[1-(3-hydroxypropyl)-1-pyrrolidinium]methyl-3-cephem-4-carboxylat

Die Herstellung erfolgt analog Beispiel 4 aus 1-(2-Aminothiazol-4-yl)-1(Z)-propencarbonsäure und 7-Amino-3-[1-(3-hydroxypropyl)-1-pyrrolidinium]methyl-3-cephem-4-carboxylat.

$^1$H-NMR (DMSO-$d_6$).

δ (ppm) = 9.26 (1H, d, J=9Hz, NH); 7,02 (2H, bs, $NH_2$); 6,34 (1H, q, J=8Hz, C=CH); 6,23 (1H, s, Thiazol); 5,70 (1H, dd, J=9Hz, J=5Hz, H-7-Lactam); 5,16 (1H, d, J=5Hz, H-6-Lactam); 5,05 (1H, d, J=13Hz, $CH_2$-Pyrrol.); 4,84 (2H, m, $CH_2$-Pyrrol., S—$CH_2$);

3,30—3,60 (9H, m, S—$CH_2$, $-CH_2-\overset{|}{\underset{|}{\overset{+}{N}}}-$, —$CH_2$—OH, Pyrrol.);

2,07 (4H, m, Pyrrol.); 1,88 (2H, m, $-CH_2$); 1,81 (3H, d, J=8Hz, C=C—$CH_3$).

Beispiel 41

(Ausgangsprodukt)

3-[1-[2-(2-Hydroxyethoxy)ethyl]-1-pyrrolidinium]-methyl-7β-phenylacetamido-3-cephem-4-carboxylat

Die Herstellung erfolgt analog Beispiel 19 aus 3-Acetoxymethyl-7β-phenylacetamido-3-cephem-4-carbonsäure und N-[2-(2-Hydroxyethoxy)ethyl]-pyrrolidin.

$^1$H-NMR ($D_6$-DMSO).

δ (ppm) = 9,15 (1H, d, J=9Hz, NH); 7,31 (5H, m, arom.); 5,57 (1H, dd, J=9Hz, J=5Hz, H-7-Lactam); 5,15

(1H, d, J=13Hz, $CH_2$-Pyrrol.); 5,08 (1H, d, J=5Hz, H-6-Lactam); 3,99 (1H, d, J=13Hz, $CH_2$-Pyrrol.); 2,90—3,90 (14H, m); 2,08 (4H, m, Pyrrol.).

Beispiel 42

7-Amino-3-[1-[2-(2-hydroxyethoxy)ethyl]-1-pyrrolidinium]-methyl-3-cephem-4-carboxylat

Die Herstellung erfolgt analog Beispiel 20 aus 3-[1-[2-(2-Hydroxyethoxy)ethyl]-1-pyrrolidinium]methyl-7β-phenylacetamido-3-cephem-4-carboxylat.

$^1$H-NMR ($D_2O$).

δ (ppm) = 5,25 (1H, d, J=5Hz, H-7-Lactam); 5,07 (1H, d, J=5Hz, H-6-Lactam); 4,65 (1H, d, J=13Hz, $CH_2$-Pyrrol.); 4,04 (1H, d, J=13Hz, $CH_2$-Pyrrol.); 3,82 (1H, d, J=18Hz, S—$CH_2$); 3,30—3,80 (9H, m); 2,05 (4H, m, Pyrrol.).

Beispiel 43

7-[1-(2-Aminothiazol-4-yl)-1(Z)-propencarboxamido]-3-[1-[2-(2-hydroxyethoxy)ethyl]-1-pyrrolidinium]-methyl-3-cephem-4-carboxylat

Die Herstellung erfolgt analog Beispiel 4 aus 1-(2-Aminothiazol-4-yl)-1(Z)-propencarbonsäure und 7-Amino-3-[1-[2-(2-hydroxyethoxy)ethyl]-1-pyrrolidinium]methyl-3-cephem-4-carboxylat.

$^1$H-NMR (DMSO-$d_6$).

δ (ppm) = 9,26 (1H, d, J=9Hz, NH); 7,03 (2H, bs, $NH_2$); 6,34 (1H, q, J=8Hz, C=CH); 6,23 (1H, s, Thiazol); 5,69 (1H, dd, J=9Hz, J=5Hz, H-7-Lactam); 5,17 (1H, d, J=5Hz, H-6-Lactam); 5,16 (1H, d, J=13Hz, $CH_2$-Pyrrol); 3,98 (1H, d, J=13Hz, $CH_2$-Pyrrol.); 3,30—3,85 (14H, m); 2,06 (4H, m, Pyrrol.); 1,81 (3H, d, J=8Hz, C=C—$CH_3$).

Beispiel 44

(Ausgangsprodukt)

3-[1-(2-Hydroxy-2-phenylethyl)-1-pyrrolidinium]methyl-7β-phenylacetamido-3-cephem-4-carboxylat

Die Herstellung erfolgt analog Beispiel 19 aus 3-Acetoxymethyl-7β-phenylacetamido-3-cephem-4-carbonsäure und DL-N-(2-Hydroxy-2-phenylethyl)pyrrolidin. Man erhält ein Gemisch aus zwei Diastereoisomeren.

$^1$H-NMR ($D_6$-DMSO).

δ (ppm) = 9,14 (1H, d, J=9Hz, NH); 7,20—7,60 (10H, m, arom.); 5,58 (1H, m, H-7-Lactam); 5,33 (1H, m, CH—OH); 5,19 (1H, m, $CH_2$-Pyrrol.); 5,07 (1H, d, J=5Hz, H-6-Lactam); 4,36 u. 4,21 (1H, d, J=13Hz, $CH_2$-Pyrrol.); 3,10—3,90 (10H, m); 2,10 (4H, m, Pyrrol.).

Beispiel 45

7-amino-3-[1-(2-hydroxy-2-phenylethyl)1-pyrrolidinium]-methyl-3-cephem-4-carboxylat

Die Herstellung erfolgt analog Beispiel 20 aus 3-[1-(2-Hydroxy-2-phenylethyl)-1-pyrrolidinium]methyl-3-cephem-4-carboxylat (Gemisch aus zwei Diastereoisomeren).

$^1$H-NMR ($D_2O$).

δ (ppm) = 7,40 (5H, bs, arom.); 5,30 (2H, m, H-7-Lactam, CH—OH); 5,14 (1H, m, H-6-Lactam); 4,82 (1H, m, $CH_2$-Pyrrol.); 4,55 u. 4,37 (1H, d, J=13Hz, $CH_2$-Pyrrol.); 3,30—4,00 (8H, m); 2,16 (4H, bs, Pyrrol.).

Beispiel 46

7-[1-(2-Aminothiazol-4-yl)-1(Z)-propencarboxamido]-3-[1-(2-hydroxy-2-phenylethyl)-1-pyrrolidinium]-methyl-3-cephem-4-carboxylat

Die Herstellung erfolgt analog Beispiel 4 aus 1-(2-Aminothiazol-4-yl)-1(Z)-propencarbonsäure und 7-Amino-3-[1-(2-hydroxy-2-phenylethyl)-1-pyrrolidinium]-methyl-3-cephem-4-carboxylat (Gemisch aus zwei Diastereoisomeren).

$^1$H-NMR (DMSO-$d_6$).

δ (ppm) = 9,24 (1H, d, J=9Hz, NH); 7,30—7,50 (5H, m, arom.); 6,98 (2H, bs, $NH_2$); 6,31 (1H, q, J=8Hz, C=CH); 6,20 u. 6,21 (1H, s, Thiazol); 5,66 (1H, m, H-7-Lactam); 5,10—5,30 (3H, m, CH—OH, $CH_2$-Pyrrol, H-6-Lactam); 4,29 u. 4,13 (1H, d, J=13Hz, $CH_2$-Pyrrol.); 3,20—3,85 (8H, m); 2,06 (4H, m, Pyrrol.); 1,77 (3H, d, J=8Hz, C=C—$CH_3$).

Beispiel 47

7-Amino-3-(diethylmethylammonium)methyl-3-cephem-4-carboxylat

Die Herstellung erfolgt analog Beispiel 5 aus 3-Chloromethyl-7β-phenylacetamido-3-cephem-4-carbonsäurebenzhydrylester.

$^1$H-NMR ($D_2O$).

δ (ppm) = 5,31 (1H, d, J=5Hz, H-7-Lactam), 5,11 (1H, d, J=5Hz, H-6-Lactam); 4,62 (1H, d, J=13Hz, $CH_2$-Ammon.); 3,88 (2H, m, $CH_2$-Ammon., S—$CH_2$); 3,48 (1H, d, J=18Hz, S—$CH_2$);

3,26 (4H, m, —$CH_2$—$\overset{|}{\overset{\pm}{N}}$—); 2,83 (3H, s, $CH_3$—$\overset{|}{\overset{\pm}{N}}$—); 1,24 (6H, m, $CH_3$).

Beispiel 48

7-[1-(2-Aminothiazol-4-yl)-1(Z)-propencarboxamido]-3-(diethylmethylammonium)methyl-3-cephem-4-carboxylat

Die Herstellung erfolgt analog Beispiel 4 aus 1-(2-Aminothiazol-4-yl)-1(Z)-propencarbonsäure und 7-Amino-3-(diethylmethylammonium)methyl-3-cephem-4-carboxylat.

$^1H$-NMR (DMSO-$D_6$).

δ (ppm) = 9,27 (1H, d, J=9Hz, NH); 7,03 (2H, bs, $NH_2$); 6,34 (1H, q, J=8Hz, C=C—H); 6,23 (1H, s, Thiazol); 5,59 (1H, dd, J=9Hz, J=5Hz, H-7-Lactam); 5,17 (1H, d, J=5Hz, H-6-Lactam); 5,11 (1H, d, J=13Hz, $CH_2$-Ammon.); 3,84 (2H, m, $CH_2$-Ammon., S—$CH_2$);

3,20—3,50 (5H, m, S—$CH_2$, $-CH_2-\overset{|}{\underset{|}{N^{\pm}}}-$); 2,86 (3H, s, $CH_3-\overset{|}{\underset{|}{N^{\pm}}}-$);

1,80 (3H, d, J=8Hz, C=C—$CH_3$); 1,24 (6H, m, $CH_2$).

Beispiel 49

7-amino-3-(triethylammonium)methyl-3-cephem-4-carboxylat

Die Herstellung erfolgt analog Beispiel 5 aus 3-Chloromethyl-7β-phenylacetamido-3-cephem-4-carbonsäurebenzhydrylester

$^1H$-NMR ($D_2O$)

δ (ppm) = 5,31 (1H, d, J=5Hz, H-7-Lactam); 5,13 (1H, d, J=5Hz, H-6-Lactam); 4,57 (1H, d, J=14Hz, $CH_2$-Ammon.); 3,89 (1H, d, J=18Hz, S—$CH_2$); 3,88 (1H, d, J=14Hz, $CH_2$-Ammon.); 3,51 (1H, d, J=18Hz, S—$CH_2$);

3,20 (6H, q, J=7Hz, $-CH_2-\overset{|}{\underset{|}{N^{\pm}}}-$); 1,21 (9H, t, J=7Hz, $CH_3$).

Beispiel 50

7-[1-(2-Aminothiazol-4-yl)-1(Z)-propencarboxamido]-3-(triethylammonium)methyl-3-cephem-4-carboxylat

Die Herstellung erfolgt analog Beispiel 4 aus 1-(2-Aminothiazol-4-yl)-1(Z)-propencarbonsäure und 7-Amino-3-(triethylammonium)methyl-3-cephem-4-carboxylat.

$^1H$-NMR (DMSO-$d_6$).

δ (ppm) = 9,29 (1H, d, J=9Hz, NH); 7,06 (2H, bs, $NH_2$); 6,36 (1H, q, J=8Hz, C=CH); 6,24 (1H, s, Thiazol); 5,70 (1H, dd, J=9Hz, J=5Hz, H-7-Lactam); 5,18 (1H, d, J=5Hz, H-6-Lactam); 5,13 (1H, d, J=13Hz, $CH_2$-Ammon); 3,83 (2H, m, $CH_2$-Ammon., S—$CH_2$);

3,20—3,50 (7H, m, S—$CH_2$, $-CH_2-\overset{|}{\underset{|}{N^{\pm}}}-$); 1,81 (3H, d, J=8Hz, C=C—$CH_3$); 1,22 (9H, m, $CH_3$).

Beispiel 51

7-Amino-3-(N,N-dimethyl-N-2-hydroxyethylammonium)methyl-3-cephem-4-carboxylat

Die Herstellung erfolgt analog Beispiel 5 aus 3-Chloromethyl-7β-phenylacetamido-3-cephem-4-carbonsäurebenzhydrylester

$^1H$-NMR ($D_2O$).

δ (ppm) = 5,35 (1H, d, J=5Hz, H-7-Lactam); 5,13 (1H, d, J=5Hz, H-6-Lactam), 4,66 (1H, d, J=13Hz, $CH_2$-Ammon.); 4,08 (1H, d, J=13Hz, $CH_2$-Ammon.); 4,00 (2H, m, $CH_2$—OH); 3,93 (1H, d, J=18Hz, S—$CH_2$); 3,51 (1H, d, J=18Hz, S—$CH_2$);

3,46 (2H, m, $-CH_2-\overset{|}{\underset{|}{N^{\pm}}}-$); 3,09 (3H, s, $CH_3-\overset{|}{\underset{|}{N^{\pm}}}-$); 3,03 (3H, s, $CH_3-\overset{|}{\underset{|}{N^{\pm}}}-$).

Beispiel 52

7-[1-(2-Aminothiazol-4-yl)-1(Z)-propencarboxamido]-3-(N,N-dimethyl-N-2-hydroxyethylammonium)-methyl-3-cephem-4-carboxylat

Die Herstellung erfolgt analog Beispiel 4 aus 1-(2-Aminothiazol-4-yl)-1(Z)-propencarbonsäure und 7-Amino-3-(N,N-dimethyl-N-2-hydroxyethylammonium)methyl-3-cephem-4-carboxylat

$^1H$-NMR (DMSO-$d_6$).

δ (ppm) = 9,31 (1H, d, J=9Hz, NH); 6,98 (2H, bs, $NH_2$); 6,31 (1H, q, J=8Hz, C=CH); 6,19 (1H, s, Thiazol); 5,65 (1H, dd, J=9Hz, J=5Hz, H-7-Lactam); 5,13 (1H, d, J=5Hz, H-6-Lactam); 5,06 (1H, d, J=13Hz, $CH_2$-Ammon.); 3,85 (4H, m, $CH_2$-Ammon., S—$CH_2$, $CH_2$—OH);

3,30—3,50 (3H, m, S—$CH_2$, —$CH_2$—±N—); 3,02 (3H, s, $CH_3$—±N—); 1,80 (3H, d, J=8Hz, C=C—$CH_3$).

### Beispiel 53

7-Amino-3-(N,N-diethyl-N-2-hydroxyethylammonium)-methyl-3-cephem-4-carboxylat

Die Herstellung erfolgt analog Beispiel 5 aus 3-Chloromethyl-7β-phenylacetamido-3-cephem-4-carbonsäurebenzhydrylester

$^1$H-NMR ($D_2O$).

δ (ppm) = 5,33 (1H, d, J=5Hz, H-7-Lactam); 5,14 (1H, d, J=5Hz, H-6-Lactam), 4,69 (1H, d, J=13Hz, $CH_2$-Ammon.); 4,05 (1H, d, J=13Hz, $CH_2$-Ammon.); 3,96 (2H, m, $CH_2$—OH); 3,89 (1H, d, J=18Hz, S—$CH_2$); 3,55 (1H, d, J=18Hz, S—$CH_2$);

3,32 (6H, m, —$CH_2$—±N—); 1,28 (6H, t, J=7Hz, $CH_3$).

### Beispiel 54

7-[1-(2-Aminothiazol-4-yl)-1(Z)-propencarboxamido]-3-(N,N-diethyl-N-2-hydroxyethylammonium)methyl-3-cephem-4-carboxylat

Die Herstellung erfolgt analog Beispiel 4 aus 1-(2-Aminothiazol-4-yl)-1(Z)-propencarbonsäure und 7-Amino-3-(N,N-diethyl-N-2-hydroxyethylammonium)methyl-3-cephem-4-carboxylat

$^1$H-NMR (DMSO-$d_6$).

δ (ppm) = 9,26 (1H, d, J=9Hz, NH); 7,01 (2H, bs, $NH_2$); 6,34 (1H, q, J=8Hz, C=CH); 6,23 (1H, s, Thiazol); 5,69 (1H, dd, J=9Hz, J=5Hz, H-7-Lactam); 5,17 (1H, d, J=5Hz, H-6-Lactam); 5,10 (1H, d, J=13Hz, $CH_2$-Ammon.); 3,92 (1H, d, J=13Hz, $CH_2$-Ammon.); 3,81 (3H, m, $CH_2OH$, S—$CH_2$),

3,30—3,50 (7H, m, S—$CH_2$, —$CH_2$—±N—); 1,81 (3H, d, J=8Hz, C=C—$CH_3$); 1,26 (6H, m, $CH_3$).

### Beispiel 55

7-Amino-3-(N,N-di-2-hydroxyethyl-N-methylammonium)methyl-3-cephem-4-carboxylat

Die Herstellung erfolgt analog Beispiel 5 aus 3-Chloromethyl-7β-phenylacetamido-3-cephem-4-carbonsäurebenzhydrylester

$^1$H-NMR ($D_2O$).

δ (ppm) = 5,34 (1H, d, J=5Hz, H-7-Lactam); 5,12 (1H, d, J=5Hz, H-6-Lactam); 4,82 (1H, d, J=13Hz, $CH_2$-Ammon.); 4,15 (1H, d, J=13Hz, $CH_2$-Ammon.); 3,97 (5H, m, S—$CH_2$, $CH_2OH$);

3,20—3,60 (5H, S—$CH_2$; —$CH_2$—±N—); 3,06, (3H, s, $CH_3$—±N—).

### Beispiel 56

7-[1-(2-Aminothiazol-4-yl)-1(Z)-propencarboxamido]-3-(N,N-di-2-hydroxyethyl-N-methylammonium)-methyl-3-cephem-4-carboxylat

Die Herstellung erfolgt analog Beispiel 4 aus 1-(2-Aminothiazol-4-yl)-1(Z)-propencarbonsäure und 7-Amino-3-(N,N-di-2-hydroxyethyl-N-methylammonium)methyl-3-cephem-4-carboxylat.

$^1$H-NMR (DMSO-$d_6$).

δ (ppm) = 9,27 (1H, d, J=9Hz, NH); 7,01 (2H, bs, $NH_2$); 6,33 (1H, q, J=8Hz, C=CH); 5,68 (1H, dd, J=9Hz, J=5Hz, H-7-Lactam); 5,17 (1H, d, J=5Hz, H-6-Lactam); 5,13 (1H, d, J=13Hz, $CH_2$-Ammon.); 4,03 (1H, d, J=12Hz, $CH_2$-Ammon.); 3,83 (5H, S—$CH_2$, $CH_2OH$);

3,20—3,60 (5H, S—$CH_2$, —$CH_2$—±N—; 3,01 (3H, s, $CH_3$—±N—); 1,80 (3H, d, J=8Hz,C=C—$CH_3$).

### Beispiel 57

7-Amino-3-(N,N-di-2-hydroxyethyl-N-ethylammonium)-methyl-3-cephem-4-carboxylat

Die Herstellung erfolgt analog Beispiel 5 aus 3-Chloromethyl-7β-phenylacetamido-3-cephem-4-carbonsäurehydrylester.

$^1$H-NMR ($D_2O$).

δ (ppm) = 5,36 (1H, d, J=5Hz, H-7-Lactam); 5,17 (1H, d, J=5Hz, H-6-Lactam), 4,84 (1H, d, J=14Hz, $CH_2$-Ammon.); 4,21 (1H, d, J=14Hz, $CH_2$-Ammon.); 3,94 (5H, m, S—$CH_2$, $CH_2OH$); 3,59 (1H, d, J=18Hz, S—$CH_2$);

3,30—3,50 (6H, m, $-CH_2-\overset{\pm}{N}-$); 1,32 (3H, m, $CH_3$).

Beispiel 58

7-[1-(2-Aminothiazol-4-yl)-1(Z)-propencarboxamido]-3-(N,N-di-2-hydroxyethyl-N-ethylammonium)methyl-3-cephem-4-carboxylat

Die Herstellung erfolgt analog Beispiel 4 aus 1-(2-Aminothiazol-4-yl)-1(Z)-propencarbonsäure und 7-Amino-3-(N,N-di-2-hydroxyethyl-N-ethylammonium)methyl-3-cephem-4-carboxylat.

$^1H$—NMR (DMSO-$d_6$).

δ (ppm) = 9,26 (1H, d, J=9Hz, NH); 7,01 (2H, bs, $NH_2$); 6,33 (1H, q, J=8Hz, C=CH); 6,21 (1H, s, Thiazol); 5,69 (1H, dd, J=9Hz, J=5Hz, H-7-Lactam); 5,16 (1H, d, J=5Hz, H-6-Lactam); 5,09 (1H, d, J=13Hz, $CH_2$-Ammon.); 4,01 (1H, d, J=13Hz, $CH_2$-Ammon.); 3,80 (5H, m, S—$CH_2$, $CH_2OH$);

3,20—3,60 (7H, m, S—$CH_2$, $-CH_2-\overset{\pm}{N}-$); 1,79 (3H, d, J=8Hz, C=C—$CH_3$); 1,26 (3H, m, $CH_3$).

Beispiel 59

7-Amino-3-(N,N-dimethyl-N-2-methoxyethylammonium)methyl-3-cephem-4-carboxylat

Die Herstellung erfolgt analog Beispiel 5 aus 3-Chloromethyl-7β-phenylacetamido-3-cephem-4-carbonsäurebenzhydrylester.

$^1H$—NMR ($D_2O$).

δ (ppm) = 5,35 (1H, d, J=5Hz, H-7-Lactam); 5,13 (1H, d, J=5Hz, H-6-Lactam); 4,68 (1H, d, J=13Hz, $CH_2$-Ammon.); 4,03 (1H, d, J=13Hz, $CH_2$-Ammon.); 3,91 (1H, d, J=18Hz, S—$CH_2$); 3,82 (2H, m, —$CH_2$—$OCH_3$);

3,50 (3H, m, S—$CH_2$, $-CH_2-\overset{\pm}{N}-$); 3,32 (3H, s, $OCH_3$); 3,05 (3H, s, $CH_3-\overset{\pm}{N}-$); 2,99 (3H, s, $CH_3-\overset{\pm}{N}-$);

Beispiel 60

7-[1-(2-Aminothiazol-4-yl)-1(Z)-propencarboxamido]-3-(N,N-dimethyl-N-2-methoxyethylammonium)-methyl-3-cephem-4-carboxylat

Die Herstellung erfolgt analog Beispiel 4 aus 1-(2-Aminothiazol-4-yl)-1(Z)-propencarbonsäure und 7-Amino-3-(N,N-dimethyl-N-2-methoxyethylammonium)methyl-3-cephem-4-carboxylat.

$^1H$—NMR (DMSO-$d_6$).

δ (ppm) = 9,26 (1H, d, J=9Hz); 7,01 (2H, bs, $NH_2$); 6.32 (1H, q, J=8Hz, C=CH); 6,21 (1H, s, Thiazol); 5,68 (1H, dd, J=9HZ, J=5Hz, H-7-Lactam); 5,17 (1H, d, J=5Hz, H-6-Lactam); 5,07 (1H, d, J=13Hz, $CH_2$-Ammon.); 3,95 (1H, d, J=13Hz, $CH_2$-Ammon.); 3,83 (1H, d, J=18Hz, S—$CH_2$); 3,78 (2H, m, —$CH_2$—$OCH_3$);

3,30—3,60 (3H, S—$CH_2$, $-CH_2-\overset{\pm}{N}-$); 3,31 (3H, s, $OCH_3$); 3,02 (3H, s, $CH_3-\overset{\pm}{N}-$); 2,97 (3H, s, $CH_3-\overset{\pm}{N}-$);

1,81 (3H, d, J=8Hz, C=C—$CH_3$).

Beispiel 61

7-Amino-3-(N-benzyl-N,N-dimethylammonium)methyl-3-cephem-4-carboxylat

Die Herstellung erfolgt analog Beispiel 5 aus 3-Chloromethyl-7β-phenylacetamido-3-cephem-4-carbonsäurebenzhydrylester.

$^1H$—NMR ($D_2O$).

δ (ppm) = 7,38 (5H, m, arom.); 5,22 (1H, d, J=5Hz, H-7-Lactam); 5,00 (1H, d, J=5Hz, H-6-Lactam); 4,55 (1H, d, J=13Hz, $CH_2$-Ammon.); 4,33 (2H, m, $CH_2$-Arom.); 3,91 (1H, d, J=13Hz, $CH_2$-Ammon.); 3,82 (1H, d, J=18Hz, S—$CH_2$); 3,34 (1H, d, J=18Hz, S—$CH_2$);

2,87 (3H, s, $CH_3-\overset{\pm}{N}-$); 2,74 (3H, s, $CH_3-\overset{\pm}{N}-$).

Beispiel 62

7-[1-(2-Aminothiazol-4-yl)-1(Z)-propencarboxamido]-3-(N-benzyl-N,N-dimethylammonium)methyl-3-cephem-4-carboxylat

Die Herstellung erfolgt analog Beispiel 4 aus 1-(2-Aminothiazol-4-yl)-1(Z)-propencarbonsäure und 7-Amino-3-(N-benzyl-N,N-dimethylammonium)methyl-3-cephem-4-carboxylat.

$^1H$—NMR (DMSO-$d_6$).

δ (ppm) = 9,29 (1H, d, J=9Hz); 7,56 (5H, m, arom.); 7,03 (2H, bs, $NH_2$); 6.35 (1H, 1, J=8Hz, C=CH); 6,24

(1H, s, Thiazol); 5,71 (1H, dd, J=9Hz, J=5Hz, H-7-Lactam); 5,23 (1H, d, J=5Hz, H-6-Lactam); 5,16 (1H, d, J=13Hz, $CH_2$-Ammon.); 4,56 (1H, d, J=12Hz, —$CH_2$-Arom.); 4,41 (1H, d, J=12Hz, —$CH_2$-Arom.); 4,03 (1H, d, J=13Hz, $CH_2$-Ammon.); 3,92 (1H, d, J=18Hz, S—$CH_2$); 3,36 (1H, d, J=18Hz, S—$CH_2$);

2,93 (3H, s, $CH_3$—$\overset{\pm}{N}$—); 2,93 (3H, s, $CH_3$—$\overset{\pm}{N}$—); 1,82 (3H, d, J=8Hz, C=C—$CH_3$).

Beispiel 63

7-Amino-3-(N,N-dimethyl-N-furfurylammonium)methyl-3-cephem-4-carboxylat

Die Herstellung erfolgt analog Beispiel 5 aus 3-Chloromethyl-7β-phenylacetamido-3-cephem-4-carbonsäurebenzhydrylester.

$^1H$—NMR ($D_2O$).

δ (ppm) = 7.65 (1H, d, J=2Hz, Furyl); 6.80 (1H, J=3Hz, Furyl); 6.54 (1H, dd, J=2Hz, J=3Hz, Furyl); 5,37 (1H, d, J=5Hz, H-7-Lactam); 5,15 (1H, d, J=5Hz, H-6-Lactam); 4,64 (1H, d, J=13Hz, $CH_2$-Ammon.); 4,54 (2H, m, $CH_2$-Furyl); 4,03 (1H, d, J=13Hz, $CH_2$-Ammon.); 3,93 (1H, d, J=18Hz, S—$CH_2$); 3,53 (1H, d, J=18Hz, S—$CH_2$);

3,03 (s, $CH_3$—$\overset{\pm}{N}$—); 2,89 (3H, s, $CH_3$—$\overset{\pm}{N}$—).

Beispiel 64

7-[1-(2-Aminothiazol-4-yl)-1(Z)-propencarboxamido]-3-(N,N-dimethyl-N-furfurylammonium)methyl-3-cephem-4-carboxylat

Die Herstellung erfolgt analog Beispiel 4 aus 1-(2-Aminothiazol-4-yl)-1(Z)-propencarbonsäure und 7-Amino-3-(N,N-dimethyl-N-furfurylammonium)methyl-3-cephem-4-carboxylat.

$^1H$—NMR (DMSO-$d_6$).

δ (ppm) = 9.27 (1H, d, J=9Hz, NH); 7,91 (1H, d, J=2Hz, Furyl); 7,02 (2H, bs, $NH_2$); 6.90 (1H, d, J=3Hz, Furyl); 6,63 (1H, dd, J=2Hz, J=3Hz, Furyl); 6,34 (1H, q, J=8Hz, C=CH; 6,23 (1H, s, Thiazol); 5,59 (1H, dd, J=9Hz, J=5Hz, H-7-Lactam); 5,21 (1H, d, J=5Hz, H-6-Lactam); 5,06 (1H, d, J=13Hz, $CH_2$-Ammon.); 4,64 (1H, d, J=13Hz, $CH_2$-Furyl); 4,52 (1H, d, J=13Hz, $CH_2$-Furyl); 3,97 (1H, d, J=13Hz, $CH_2$-Ammon.); 3,87 (1H, d, J=18Hz, S—$CH_2$); 3,34 (1H, d, J=18Hz, S—$CH_2$);

2,95 (3H, s, $CH_3$—$\overset{\pm}{N}$—); 3,87 (3H, s, $CH_3$—$\overset{\pm}{N}$—).

Beispiel 65

7-Amino-3-(N,N-dimethyl-N-3-formamidopropylammonium)methyl-3-cephem-4-carboxylat

Die Herstellung erfolgt analog Beispiel 5 aus 3-Chloromethyl-7β-phenylacetamido-3-cephem-4-carbonsäurebenzhydrylester.

$^1H$—NMR ($D_2O$).

δ (ppm) = 8,04 (1H, s, CHO); 5,41 (1H, d, J=5Hz, H-7-Lactam); 5,18 (1H, d, J=5Hz, H-6-Lactam); 4,68 (1H, d, J=13Hz, $CH_2$-Ammon.); 4,03 (1H, d, J=13Hz, $CH_2$-Ammon.); 3,95 (1H, d, J=18Hz, S—$CH_2$); 3.54 (1H, d, J=18Hz, S—$CH_2$); 3,10—3,40 (4H, m);

3,04 (3H, s, $CH_3$—$\overset{\pm}{N}$—); 2,99 (3H, s, $CH_3$—$\overset{\pm}{N}$—); 1,98 (2H, m).

Beispiel 66

7-[1-(2-Aminothiazol-4-yl)-1-(Z)-propencarboxamido]-3-(N,N-dimethyl-N-3-formamidopropylammonium)-methyl-3-cephem-4-carboxylat

Die Herstellung erfolgt analog Beispiel 4 aus 1-(2-Aminothiazol-4-yl)-1(Z)-propencarbonsäure und 7-Amino-3-(N,N-dimethyl-3-formamidopropylammonium)methyl-3-cephem-4-carboxylat.

$^1H$—NMR (DMSO-$d_6$).

δ (ppm) = 9,26 (1H, d, J=9Hz, NH); 8,05 (1H, s, CHO); 7,02 (2H, bs, $NH_2$); 6,34 (1H, q, J=8Hz, C=CH); 6,22 (1H, s, Thiazol); 5,69 (1H, dd, J=9Hz, J=5Hz, H-7-Lactam); 5,19 (1H, d, J=5Hz, H-6-Lactam); 5,05 (1H, d, J=13Hz, $CH_2$-Ammon.); 4,90 (2H, m, $CH_2$-Ammon., S—$CH_2$); 3,10—3,50 (5H, m);

2,97 (3H, s, $CH_3$—$\overset{\pm}{N}$—); 2,92 (3H, s, $CH_3$—$\overset{\pm}{N}$—); 1,90 (2H, m, —$CH_2$); 1,79 (3H, d, J=8Hz, C=C—$CH_3$).

Beispiel 67

7-Amino-3-(4-hydroxy-1-methylpiperidinium)methyl-3-cephem-4-carboxylat

Unter Stickstoff werden 1,56 g (4 mmol) 3-Acetoxymethyl-7β-phenylacetamido-3-cephem-4-carbonsäure bei Raumtemperatur in 16 ml absol. Methylenchlorid aufgeschlämmt und durch Zugabe von 2,56 ml (12 mmol) N-Methyl-N-trimethylsilyltrifluoracetamid (MSTFA) in Lösung gebracht. Nach Abkühlen auf 0°C werden 8 ml einer 2 molaren Lösung von Trimethylsilyljodid in Methylenchlorid zugegeben und die Reaktionslösung wird 1 Stunde bei 0°C gerührt. Nach Zugabe von 2,52 ml (30,8 mmol) absol. Tetrahydrofuran wird weitere 15 Minuten bei 0°C gerührt. Anschließend werden 2,3 g (20 mmol) 4-Hydroxy-N-methylpiperidin zugegeben und die Lösung wird 30 Minuten nachgerührt. Dann werden 0,8 ml Wasser und nach weiteren 5 Minuten 100 ml absol. Ether zugegeben. Der Ether wird abdekantiert, der Rückstand nochmals mit Ether verrührt und nach erneutem Dekantieren wird der Rückstand durch Zugabe von $NaHCO_3$ in 50 ml Wasser gelöst. Anschließend werden 4 g Penicillin-G-Acylase zugegeben und der pH-Wert wird durch Zugabe von 4N-Triethylamin in Ethanol konstant bei 7,8 gehalten. Nach Beendigung der enzymatischen Spaltung wird von der Acylase abfiltriert und das Filtrat mit konz. Salzsäure auf pH 2 gestellt. Vom entstehenden Niederschlag wird über Kieselgel abgesaugt und das Filtrat wird zu 2 l Aceton zugetropft. Das gewünschte Produkt fällt als Hydrochlorid aus und wird abgesaugt und getrocknet.

Man erhält 875 mg (60%) gewünschtes Produkt als Gemisch von zwei Diastereoisomeren.

$^1H$—NMR ($D_2O$).

δ (ppm) = 5,49 (1H, d, J=5Hz, H-7-Lactam); 5,26 (1H, d, J=5Hz, H-6-Lactam); 4,80 (1H, m, $CH_2$-Pip.); 4,16 (2H, m, $CH_2$-Pip., CH—OH); 4,04 (1H, d, J=18Hz, S—$CH_2$); 3,64 (1H, d, J=18Hz, S—$CH_2$); 3,55 (2H, m, Pip.); 3,38 (2H, m, Pip.);

3,11 u. 3,08 (3H, s, $CH_3$—$\overset{+}{N}$—); 2,20 (2H, m, Pip); 1,95 (2H, m, Pip.).

Beispiel 68

7-[1-(2-Aminothiazol-4-yl)-1(Z)-propencarboxamido]-3-(4-hydroxy-1-methylpiperidinium)methyl-3-cephem-4-carboxylat

Die Herstellung erfolgt analog Beispiel 4 aus 1-(2-Aminothiazol-4-yl)-1(Z)-propencarbonsäure und dem in Beispiel 67 erhaltenen Diastereoisomerengemisch.

$^1H$—NMR (DMSO-$d_6$).

δ (ppm) = 9,27 (1H, d, J=9Hz, NH); 7.03 (2H, bs, $NH_2$); 6,36 (1H, q, J=8Hz, C=CH); 6,24 (1H, s, Thiazol); 5,70 (1H, dd, J=9Hz, J=5Hz, H-7-Lactam), 5,19 (1H, d, J=5Hz, H-6-Lactam); 5,01 (1H, d, J=13Hz, $CH_2$-Pip.); 3,99 (1H, d, J=13Hz, $CH_2$-Pip.); 3,85 (2H, m, S—$CH_2$, CH—OH); 3,20—3,50 (5H, m, S—$CH_2$, Pip.);

2,99 u. 2,97 (3H, s, $CH_3$—$\overset{+}{N}$—); 2,02 (2H, m, Pip.); 1.83 (2H, d, J=8Hz, C=C—$CH_3$); 1,77 (2H, m, Pip.).

Beispiel 69

7-Amino-3-(4-hydroxymethyl-1-methylpiperidinium)methyl-3-cephem-4-carboxylat

Die Herstellung erfolgt analog Beispiel 67 aus 3-Acetoxymethyl-7β-phenylacetamido-3-cephem-4-carbonsäure und 4-Hydroxymethyl-N-methylpiperidin.

$^1H$—NMR ($D_2O$).

δ (ppm) = 5,30 [1]d, J=5Hz, 5,10 [1]d, J=5Hz, 4,60 [1]d, J=13Hz, 4,01 [1]d, J=13Hz, 3,88 [1]d, J=18Hz, 3,10—3,65 [7]m, 2,92 [3]s, 1,40—1,95 [5]m.

Beispiel 70

7-[1-(2-Aminothiazol-4-yl)-1(Z)-propencarboxamido]-3-(4-hydroxymethyl-1-methylpiperidinium)methyl-3-cephem-4-carboxylat

Die Herstellung erfolgt analog Beispiel 4 aus 1-(2-Aminothiazol-4-yl)-1(Z)-propencarbonsäure und 7-Amino-3-(4-hydroxymethyl-1-methylpiperidinium)methyl-3-cephem-4-carboxylat.

$^1H$—NMR (DMSO-$d_6$).

δ (ppm) = 9,27 [1]d, J=9Hz, 7,03 [2]bs, 6,35 [1]q, J=8Hz, 6,34 [1]s, 5,69 [1]dd, J=9Hz, J=5Hz, 5,18 [1]d, J=5Hz, 5,02 [1]d, J=13Hz, 4,00 [1]d, J=13Hz, 3,81 [1]d, J=17Hz, 3,2—3,6 [8]m, 2,95 [3]s, 1,81 [3]d, J=8Hz, 1,50—1,95 [5]m.

Beispiel 71

7-Amino-3-(4-formylaminomethyl-1-methylpiperidinium)methyl-3-cephem-4-carboxylat

Die Herstellung erfolgt analog Beispiel 67 aus 3-Acetoxymethyl-7β-phenylacetamido-3-cephem-4-carbonsäure und 4-Formylaminomethyl-N-methylpiperidin.

$^1H$—NMR ($D_2O$).

δ (ppm) = 8.09 [1]d, J=6Hz, 5,18 [1]d, J=6Hz, 4,65 [1]d, J=13Hz, 4,11 [1]d, J=13Hz, 3,95 [1]d, J=18Hz, 3,20—3,75 [7]m, 3,03 [3]s, 1,60—2,10 [5]m.

Beispiel 72

7-[1-(2-Aminothiazol-4-yl)-1(Z)-propencarboxamido]-3-(4-formylaminomethyl-1-methylpiperidinium)-methyl-3-cephem-4-carboxylat

Die Herstellung erfolgt analog Beispiel 4 aus 1-(2-Aminothiazol-4-yl)-1(Z)-propencarbonsäure und 7-Amino-2-(4-formylaminomethyl-1-methylpiperidinium)methyl-3-cephem-4-carboxylat.

$^1$H—NMR (DMSO-$d_6$).

δ (ppm) = 9,27 [1]d, J=9Hz, 8,26 [1]bs, 8,08 [2]s, 7,02 [2]s, 6,34 [1]q, J=8Hz, 6,24 [1]s, 5,70 [1]dd, J=9Hz, J=5Hz, 5,18 [1]d, J=5Hz, 5,01 [1]d, J=13Hz, 4,06 [1]d, J=13Hz, 3,81 [1]d, J=17Hz, 3,00—3,60 [7]m, 2,93 [3]s, 1,80 [3]d, J=8Hz, 1,75 [5]m.

Beispiel 73

7-Amino-3-(4-aminocarbonyl-1-methylpiperidinium)methyl-3-cephem-4-carboxylat

Die Herstellung erfolgt analog Beispiel 67 aus 3-Acetoxymethyl-7β-phenylacetamido-3-cephem-4-carbonsäure und 4-Aminocarbonyl-N-methylpiperidin.

$^1$H—NMR ($D_2O$).

δ (ppm) = 5,36 [1]d, J=5Hz, 5,14 [1]d, J=5Hz, 4,72 [1]d, J=13Hz, 4,17 [1]d, J=13Hz, 3,97 [1]d, J=16Hz, 3,56 [1]d, J=16Hz, 3,03 [3]s, 2,73 [1]m, 2,14 [4].

Beispiel 74

7-[1-(2-Aminothiazol-4-yl)-1(Z)-propencarboxamido]-3-(4-aminocarbonyl-1-methylpiperidinium)methyl-3-cephem-4-carboxylat

Die Herstellung erfolgt analog Beispiel 4 aus 1-(2-Aminothiazol-4-yl)-1(Z)-propencarbonsäure und 7-Amino-3-(4-aminocarbonyl-1-methylpiperidinium)methyl-3-cephem-4-carboxylat.

$^1$H—NMR (DMSO-$d_6$).

δ (ppm) = 9,24 [1]d, J=9Hz, 7,47 [2]bs, 6,33 [1]q, J=8Hz, 6,22 [1]s, 5,67 [1]dd, J=9Hz, J=5Hz, 5,15 [1]d, J=5Hz, 5,00 [1]d, J=12Hz, 3,99 [1]d, J=12Hz, 3,80 [1]d, J=18Hz, 3,20—3,60 [5]m, 2,96 [3]s, 2,42 [1]m, 1,83—2,30 [4]m, 1,82 [3]d, J=8Hz.

Beispiel 75

7-Amino-3-(3-hydroxy-1-methylpiperidinium)methyl-3-cephem-4-carboxylat

Die Herstellung erfolgt analog Beispiel 67 aus 3-Acetoxymethyl-7β-phenylacetamido-3-cephem-4-carbonsäure und 3-Hydroxy-N-methylpiperidin.

Man erhält ein Gemisch aus vier Diastereoisomeren.

Beispiel 76

7-[1-(2-Aminothiazol-4-yl)-1(Z)-propencarboxyamido]-3-(3-hydroxy-1-methylpiperidinium)methyl-3-cephem-4-carboxylat

Die Herstellung erfolgt analog Beispiel 4 aus dem in Beispiel 75 erhaltenen Diastereomerengemisch und 1-(2-Aminothiazol-4-yl)-1(Z)-propencarbonsäure.

Man erhält ein Gemisch aus vier Diastereoisomeren A—D im Verhältnis A:B:C:D = 1:2:1:2 (nach HPLC).

Beispiel 77

7-Amino-3-(2-hydroxymethyl-1-methylpiperidinium)methyl-3-cephem-4-carboxylat

Die Herstellung erfolgt analog Beispiel 67 aus 3-Acetoxymethyl-7β-phenylacetamido-3-cephem-4-carbonsäure und 2-Hydroxymethyl-N-methylpiperidin.

Man erhält ein Gemisch aus vier Diastereoisomeren.

Beispiel 78

7-[1-(2-Aminothiazol-4-yl)-1(Z)-propencarboxamido]-3-(2-hydroxymethyl-1-methylpiperidinium)methyl-3-cephem-4-carboxylat

Die Herstellung erfolgt analog Beispiel 4 aus dem in Beispiel 77 erhaltenen Diastereoisomerengemisch und 1-(2-Aminothiazol-4-yl)-1(Z)-propencarbonsäure.

Man erhält ein Gemisch aus vier Diastereoisomeren A—D im Verhältnis A:B:C:D = 10:1:1:10.

Das Gemisch kann durch Reverse-Phase-Chromatographie getrennt werden.

Beispiel 79

7-Amino-3-[4-(3-hydroxypropyl]-1-methylpiperazinium)methyl-3-cephem-4-carboxylat

Die Herstellung erfolgt analog Beispiel 67 aus 3-Acetoxymethy-7β-phenylacetamido-3-cephem-4-carbonsäure und N-(3-Hydroxypropyl)-N′-methylpiperazin.

$^1$H—NMR ($D_2O$).

δ (ppm) = 5,48 (1H, d, J=5Hz, H-7-Lactam); 5,27 (1H, d, J=5Hz, H-6-Lactam); 4.92 (1H, d, J=13Hz, $CH_2$-Pip.); 4.37 (1H, d, J=13Hz, $CH_2$-Pip.); 4,02 (1H, d, J=18Hz, S—$CH_2$); 3,40—4,00 (13H, m);

3,32 (3H, s, $CH_3$—$\overset{+}{\underset{|}{\overset{|}{N}}}$—); 2,04 (2H, m, —$CH_2$).

Beispiel 80

7-[1-(2-Aminothiazol-4-yl)-1(Z)-propencarboxamido]-3-[4-(3-hydroxypropyl)-1-methylpiperazinium]methyl-3-cephem-4-carboxylat

Die Herstellung erfolgt analog Beispiel 4 aus 1-(2-Aminothiazol-4-yl)-1(Z)-propencarbonsäure und 7-Amino-3-[4-(3-hydroxypropyl)-1-methylpiperazinium]methyl-3-cephem-4-carboxylat.

$^1$H—NMR (DMSO-$d_6$).

δ (ppm) = 9,27 (1H, d, J=9Hz, NH); 7,03 (1H, bs, $NH_2$); 6,33 (1H, g, J=8Hz, C=CH); 6,22 (1H, s, Thiazol); 5,56 (1H, dd, J=9Hz, J=5Hz, H-7-Lactam); 5,17 (1H, d, J=13Hz, $CH_2$-Pip.); 4,00 (1H, d, J=13Hz, $CH_2$-Pip.); 3,83 (1H, d, J=18Hz, S—$CH_2$); 3,20—3,50 (7H, m);

2,97 (3H, s, $CH_3$—$\overset{+}{-}$N—);

2,79 (2H, m); 2,45 (2H, m); 1,79 (3H, d, J=8Hz, C=C—$CH_3$); 1,56 (2H, m, —$CH_2$).

Beispiel 81

7-Amino-3-(4-formyl-1-methylpiperazinium)methyl-3-cephem-4-carboxylat

Die Herstellung erfolgt analog Beispiel 67 aus 3-Acetoxymethyl-7β-phenylacetamido-3-cephem-4-carbonsäure und N-Formyl-N'-methylpiperazin.

$^1$H—NMR ($D_2O$).

δ (ppm) = 8,02 [1]s, 5,38 [1]d, J=5Hz, 5,17 [1]d, J=5Hz, 4,78 [1]d, J=13Hz, 4,14 [2]d, J=13Hz, 3,70—3,98 [3]m, 3,30—3,64 [6]m, 3,13 [3]s.

Beispiel 82

7-[1-(2-Aminothiazol-4-yl)-1(Z)-propencarboxamido]-3-(4-formyl-1-methylpiperazinium)methyl-3-cephem-4-carboxylat

Die Herstellung erfolgt analog Beispiel 4 aus 1-(3-Aminothiazol-4-yl)-1(Z)-propensäure und 7-Amino-4-(4-formyl-1-methylpiperazinium)methyl-3-cephem-4-carboxylat.

$^1$H—NMR (DMSO-$d_6$).

δ (ppm) = 9,25 [1]d, J=9Hz, 8,08 [1]s, 7,03 [1]q, J=8Hz, 6,23 [1]s, 5,67 [1]dd, J=9Hz, J=5Hz, 5,17 [1]d, J=5Hz, 5,15 [1]d, J=13Hz, 4,03 [1]d, J=13Hz, 3,20—3,95 [10]m, 3,08 [3]s, 1,89 [3]d, J=8Hz.

Beispiel 83

7-Amino-3-(4-aminocarbonyl-1-methylpiperazinium)methyl-3-cephem-4-carboxylat

Die Herstellung erfolgt analog Beispiel 67 aus 3-Acetoxymethyl-7β-phenylacetamido-3-cephem-4-carbonsäure und N-Aminocarbonyl-N'-methylpiperazin.

$^1$H—NMR ($D_2O$).

δ (ppm) = 5,39 [1]d, J=5Hz, 5,16 [1]d, J=5Hz, 4,80 [1]d, J=13Hz, 4,14 [1]d, J=13Hz, 3,85—4,02 [3]m, 3,35—3,72 [7]m, 3,22 [3]s.

Beispiel 84

7-[1-(2-Aminothiazol-4-yl)-1(Z)-propencarboxamido]-3-(4-aminocarbonyl-1-methylpiperazinium)methyl-3-cephem-4-carboxylat

Die Herstellung erfolgt analog Beispiel 4 aus 1-(2-Aminothiazol-4yl)-1(Z)-propencarbonsäure und 7-Amino-3-(4-aminocarbonyl-1-methylpiperazinium)methyl-3-cephem-4-carboxylat.

$^1$H—NMR (DMSO-$d_6$).

δ (ppm) = 9,24 [1]d, J=9Hz, 6,99 [2]bs, 6,31 [1]q; J=8Hz, 6,30 [2]bs, 6,20 [1]s, 5,67 [1]dd, J=9Hz, J=5Hz, 5,15 [1]d, J=5Hz, 5,09 [1]d, J=13Hz, 3,99 [1]d, J=13Hz, 3,78 [3]m, 3,10—3,65 [7]m, 3,00 [3]s, 1,78 [3]d, J=8Hz.

Beispiel 85

7-Amino-3-(4-methylsulfonyl-1-methylpiperazinium)methyl-3-cephem-4-carboxylat

Die Herstellung erfolgt analog Beispiel 67 aus 3-Acetoxymethyl-7β-phenylacetamido-3-cephem-4-carbonsäure und N-Methylsulfonyl-N'-methylpiperazin.

$^1$H—NMR ($D_2O$).

δ (ppm) = 5,39 [1]d, J=5Hz, 5,17 [1]d, J=5Hz, 4,78 [1]d, J=13Hz, 4,14 [1]d, J=13Hz, 3,92 [1]d, J=18Hz, 3,40—3,80 [9]m, 3,10 [3]s, 3,06 [3]s.

Beispiel 86

7-[1-(2-Aminothiazol-4-yl)-1(Z)-propencarboxamido]-3-(4-methylsulfonyl-1-methylpiperazinium)methyl-3-cephem-4-carboxylat

Die Herstellung erfolgt analog Beispiel 4 aus 1-(2-Aminothiazol-4-yl)-1(Z)-propencarbonsäure und 7-Amino-3-(4-methylsulfonyl-1-methylpiperazinium)methyl-3-cephem-4-carboxylat.

$^1$H—NMR (DMSO-$d_6$).

δ (ppm) = 9,10 [1]d, J=9Hz, 7,02 [2]bs, 6,33 [1]q, J=8Hz, 6,21 [1]s, 5,69 [1]dd, J=9Hz, J=5Hz, 5,15 [1]d, J=5Hz, 5,13 [1]d, J=13Hz, 4,08 [1]d, J=13Hz, 3,83 [1]d, J=18Hz, 3,20—3,73 [9]m, 3,05 [3]s, 3,03 [3]s, 1,80 [3]d, J=8Hz.

Beispiel 87

7-Amino-3-(4-dimethylaminosulfonyl-1-methylpiperazinium)methyl-3-cephem-4-carboxylat

Die Herstellung erfolgt analog Beispiel 67 aus 3-Acetoxymethyl-7β-phenylacetamido-3-cephem-4-carbonsäure und N-Dimethylaminosulfonyl-N'-methylpiperazin.

$^1H$—NMR ($D_2O$).

δ (ppm) = 5,39 [1]d, J=5Hz, 5,16 [1]d, J=5Hz, 4,82 [1]d, J=13Hz, 4.18 [1]d, J=13Hz, 3,98 [1]d, J=18Hz, 3,48—3,80 [9]m, 3,13 [3]s, 2,89 [6]s.

Beispiel 88

7-[1-(2-Aminothiazol-4-yl)-1(Z)-propencarboxamido]-3-(4-dimethylaminosulfonyl-1-methylpiperazinium)-methyl-3-cephem-4-carboxylat

Die Herstellung erfolgt analog Beispiel 4 aus 1-(2-Aminothiazol-4-yl)-(Z)-propencarbonsäure und 7-Amino-3-(4-dimethylaminosulfonyl-1-methylpiperazinium)methyl-3-cephem-4-carboxylat.

$^1H$—NMR (DMSO-$d_6$).

δ (ppm) = 9,25 [1]d, J=9Hz, 7,00 [2]bs, 6,32 [1]q, J=8Hz, 6,20 [1]s, 5,68 [1]dd, J=9Hz, J=5Hz, 5,16 [1]d, J=5Hz, 5,14 [1]d, J=13Hz, 4,05 [1]d, 3,81 [1]d, J=18Hz, 3,20—3,70 [9]m, 3,04 [3]s, 2.80 [6]s.

Beispiel 89

7-Amino-3-(1,4-dimethyl-3-oxopiperazinium)methyl-3-cephem-4-carboxylat

Die Herstellung erfolgt analog Beispiel 67 aus 3-Acetoxymethyl-7β-phenylacetamido-3-cephem-4-carbonsäure und 1,4-Dimethyl-3-oxopiperazin. Man erhält ein Gemisch aus 2 Isomeren.

$^1H$—NMR ($D_2O$).

δ (ppm) = 5,40 und 5,39 [1]d, J=5Hz, 5,19 und 5,18 [1]d, J=5Hz, 4,86 und 4,76 [1]d, J=13Hz, 4,24 und 4,21 [1]d, J=13Hz, 4,24 [1]d, J=18Hz, 4,06 [1]m, 3,62—4,00 [5]m, 3,56 und 3,54 [1]d, J=18Hz, 3,17 und 3,15 [3]s, 2,98 [3]s..

Beispiel 90

7-[1-(2-Aminothiazol-4-yl)-1(Z)-propencarboxamido]-3-(1,4-dimethyl-3-oxopiperazinium)methyl-3-cephem-4-carboxylat

Die Herstellung erfolgt analog Beispiel 4 aus 1-(2-Aminothiazol-4-yl)-1(Z)-propencarbonsäure und 7-Amino-3-(1,4-dimethyl-3-oxopiperazinium)methyl-3-cephem-4-carboxylat (Gemisch aus 2 Isomeren).

$^1H$—NMR (DMO-$d_6$).

δ (ppm) = 9,29 [1]bd, J=9Hz, 7,02 [2]bs, 6,35 [1]q. J=8Hz, 6,24 [1]s, 5,72 [1]dd, J=9Hz, J=5Hz, 5,19 und 5,18 [1]d, J=5Hz, 5,08 und 5,04 [1]d, J=13Hz, 4,33 und 4,12 [1]d, J=13Hz, 3,20—4,20 [8]m, 3,08 [3]bs, 2,92 [3]bs, 1,82 [3]d, J=8Hz.

**Patentansprüche**

1. β-Lactamverbindungen der allgemeinen Formel (I)

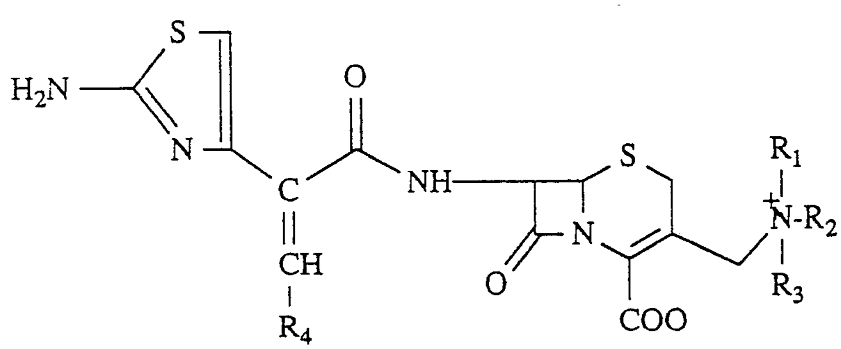

(I)

worin

$R_1$, $R_2$ und $R_3$ gleich oder verschieden voneinander sind und einen $C_1$—$C_6$-Alkylrest, der gegebenenfalls mit einem oder zwei Substituenten aus der Gruppe Hydroxy, Carboxy, $C_1$—$C_6$-Alkyloxycarbonyl, Formyl oder $C_1$—$C_6$-Alkylcarbonyl, Carbamoyl, Sulfo, Cyano, Nitro, Amino, Halogen, $C_1$—$C_6$-Alkyl- und Dialkylamino, $C_1$—$C_6$-Alkylcarbonylamino, $C_1$—$C_6$-Alkyloxy, $C_1$—$C_6$-Alkylthio, $C_1$—$C_6$-Alkylsulfinyl, $C_1$—$C_6$-Alkylsulfonyl, Phenyl, Naphthyl oder Pyridyl substituiert ist, oder einen 3- bis 7-gliedrigen Ring darstellen, oder $R_1$ die obige Bedeutung hat und $R_2$ und $R_3$ gemeinsam mit dem $N^+$-Atom einen gegebenenfalls mit

einem oder zwei Substituenten aus der Gruppe $C_1$—$C_6$-Alkyl, Hydroxy, Hydroxy-$C_1$—$C_6$-Alkyl, Carboxy, $C_1$—$C_6$-Alkyloxycarbonyl, Formyl oder $C_1$—$C_6$-Alkylcarbonyl, Carbamoyl, Sulfo, Cyano, Nitro, Amino, Halogen, $C_1$—$C_6$-Alkyl- und Dialkylamino, $C_1$—$C_6$-Alkylcarbonylamino, $C_1$—$C_6$-Alkyloxy, $C_1$—$C_6$-Alkylthio, $C_1$—$C_6$-Alkylsulfinyl oder $C_1$—$C_6$-Alkylsulfonyl substituierten 3- bis 7-gliedrigen carbo- oder heterocyclischen Ring, bilden, der gesättigt oder ungesättigt sein kann und bis zu drei Heteroatome enthalten kann, die Sauerstoff, Stickstoff und/oder Schwefel sein können, und

$R_4$ Wasserstoff, einen gegenbenenfalls geradkettigen oder verzweigten Alkylrest mit bis zu 6 C-Atomen, der auch 1 oder 2 Doppelbindungen enthalten und carbocyclisch sein kann, einen Arylrest der Formel

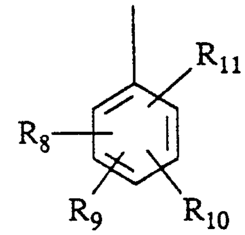

worin

$R_8$, $R_9$, $R_{10}$ und $R_{11}$ unabhängig voneinander Wasserstoff, F, Cl, Br, $C_1$—$C_6$-Alkyl, Phenyl, eine Gruppe —$OCOR_{12}$, eine Gruppe

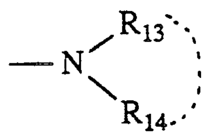

Nitro, Cyano, $C_1$—$C_4$-Alkoxy, $C_1$—$C_4$-Alkylthio, Hydroxycarbonyl, $C_1$—$C_4$-Alkoxycarbonyl, Aminocarbonyloxy, Hydroxycarbonyl-$C_1$—$C_6$-Alkyl, $C_1$—$C_4$-Alkoxycarbonyl-$C_1$—$C_6$-Alkyl, Sulfonyl oder Sulfo bedeutet, und worin $R_{12}$ $C_1$—$C_6$-Alkyl bedeutet, und worin $R_{13}$ und $R_{14}$ unabhängig voneinander Wasserstoff, oder gemeinsam oder unabhängig voneinander $C_1$—$C_6$-Alkyl oder $C_1$—$C_6$-Alkanoyl sein können,

eine 5- oder 6-Ring mit 1—4 Heteroatomen, gleich oder verschieden aus der Gruppe N, O und S, oder Hydroxycarbonyl, Hydroxycarbonyl-$C_1$—$C_4$-alkyl, $C_1$—$C_6$-Alkoxycarbonyl, Halogen oder Pseudohalogen bedeutet.

2. β-Lactamverbindungen nach Anspruch 1, in denen der 3- bis 7-gliedrige carbo- oder heterocyclische Ring ein oder zwei Heteroatome enthalten kann.

3. Verfahren zur Herstellung von Verbindungen der allgemeinen Formel (I) nach Anspruch 1

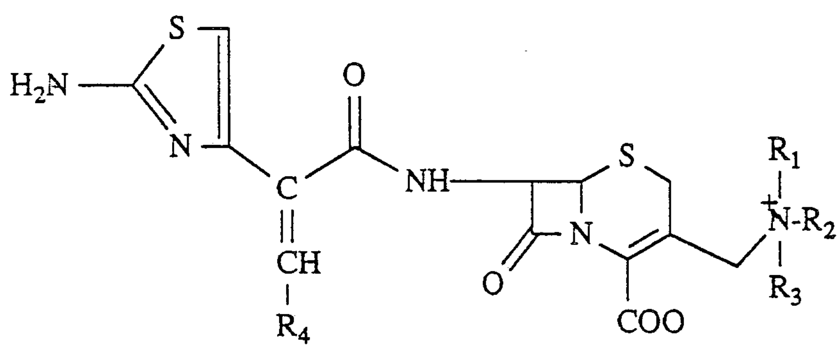

(I)

dadurch gekennzeichnet, daß man Verbindungen der allgemeinen Formel (III)

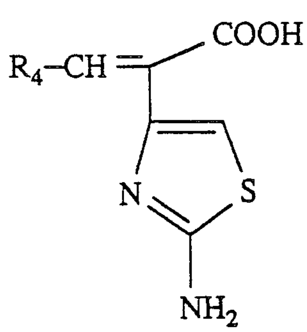

(III)

worin

$R_4$ die in Anspruch 1 angegebene Bedeutung hat, in denen die Aminogruppe geschützt oder ungeschütst vorliegen kann, nach Aktivierung der Carboxylgruppe durch Überführung in ein gemischtes Anhydrid, insbesondere mit Chlorameisensäureethylester oder Methansulfonsäurechlorid, nach Überührung in das Säaurehalogenid oder nach Überführung in einen aktivierten Ester mit insbesondere N-Hydroxybenztriazol und Dicyclohexylcarbodiimid, mit Verbindungen der allgemeinen Formel (IV),

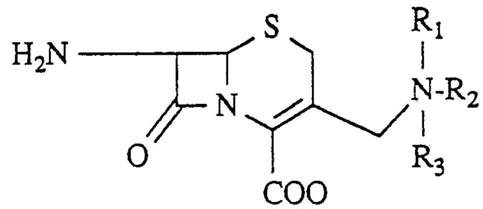
(IV)

worin

$R_1$, $R_2$ und $R_3$ die in Anspruch 1 gennante Bedeutung besitzen, zur Umsetzung bringt, dann gegebenenfalls die Schutzgruppen abspaltet und die gewünschten Salze oder aus Salzen die freien Säuren herstellt.

4. Verbindungen der allgemeinen Formel (IV)

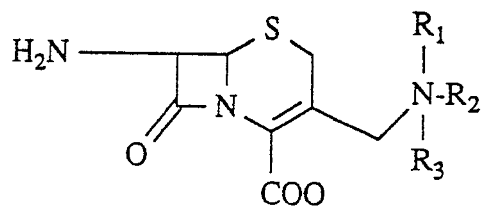
(IV)

in denen

$R_1$, $R_2$ und $R_3$ gleich oder verschieden voneinander sind und einen $C_1$—$C_6$-Alkylrest oder einen 3- bis 7-gliedrigen Ring darstellen, oder $R_1$ die obige Bedeutung hat und $R_2$ und $R_3$ gemeinsam mit dem $N^+$-Atom einen 3- bis 7-gliedrigen Ring bilden, der gesättigt oder ungesättigt sein kann und ein oder zwei weitere Heteroatome enthalten kann, die Sauerstoff, Stickstoff und/oder Schwefel sein können.

5. Arzneimittel enthaltend mindestens eine Verbindung nach einem der Ansprüche 1 und 2.

6. Verwendung der Verbindungen gemäß einem der Ansprüche 1 und 2 zur Herstellung von Arzneimitteln.

7. Verwendung von β-Lactamverbindungen der Formel (I) nach Anspruch 1

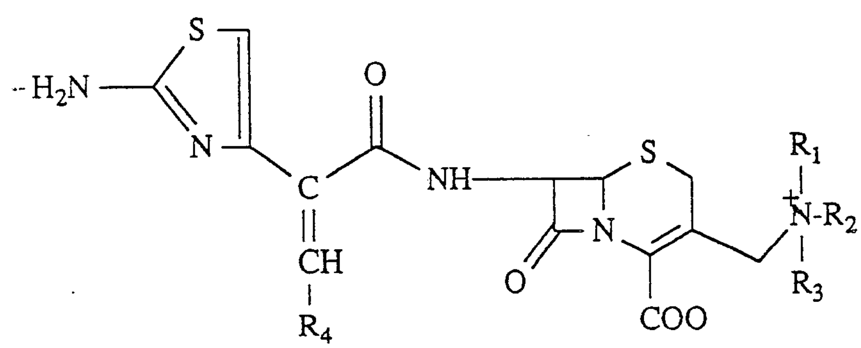
(I)

worin

$R_1$, $R_2$, $R_3$ und $R_4$ die im Anspruch 1 angegebene Bedeutung haben, als Wachstumsförderer für Tiere.

**Revendications**

1. Composés de β-lactame de formule générale

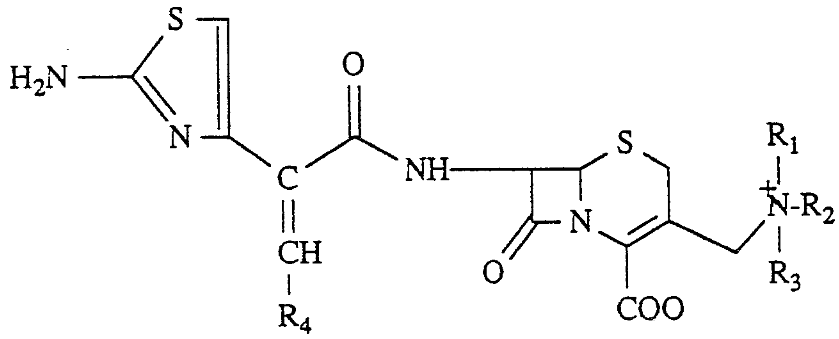

(I)

dans laquelle

$R_1$, $R_2$ et $R_3$ sont identiques ou sont différents les uns des autres et représentent un reste alkyle en $C_1$ à $C_6$, qui est substitué le cas échéant avec un ou deux substituants choisis dans le groupe des radicaux hydroxy, carboxy, (alkyloxy en $C_1$ à $C_6$)-carbonyle, formyle ou (alkyle en $C_1$ à $C_6$)-carbonyle, carbamoyle, sulfo, cyano, nitro, amino, halogéno, alkylamino en $C_1$ à $C_6$ et di-(alkyle en $C_1$ à $C_6$)-amino, (alkyle en $C_1$ à $C_6$)-carbonylamino, alkyloxy en $C_1$ à $C_6$, alkylthio en $C_1$ à $C_6$, alkylsulfinyle en $C_1$ à $C_6$, alkylsulfonyle en $C_1$ à $C_6$, phényle, naphtyle ou pyridyle, ou représentent un noyau de trois à sept chaînons, ou bien $R_1$ à la définition indiquée ci-dessus et $R_2$ et $R_3$ forment conjointement avec l'atome $N^+$ un noyau carbocyclique ou hétérocyclique de trois à sept chaînons, portant éventuellement 1 ou 2 substituants choisis dans le groupe des radicaux alkyle en $C_1$ à $C_6$, hydroxy, hydroxy (alkyle en $C_1$ à $C_6$), carboxy, (alkoxy en $C_1$ à $C_6$)-carbonyle, formyle ou (alkyle en $C_1$ à $C_6$)-carbonyle, carbamoyle, sulfo, cyano, nitro, amino, halogéno, alkylamino en $C_1$ à $C_6$ et di-(alkyle en $C_1$ à $C_6$)-amino, (alkyle en $C_1$ à $C_6$)-carbonylamino, alkyloxy en $C_1$ à $C_6$, alkylthio en $C_1$ à $C_6$, alkylsulfinyle en $C_1$ à $C_6$ ou alkylsulfonyle en $C_1$ à $C_6$, noyau qui peut être saturé ou non saturé et qui peut comporter jusqu'à trois hétéro-atomes qui peuvent êre des atomes d'oxygène, d'azote et/ou de soufre, et

$R_4$ représente l'hydrogène, un reste alkyle, le cas échéant à chaîne droite ou ramifié ayant jusqu'à 6 atomes de carbone, qui peut comporter une ou deux doubles liaisons et qui peut être carbocyclique, un reste aryle de formule

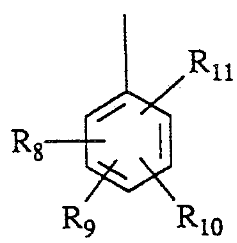

dans laquelle

$R_8$, $R_9$, $R_{10}$ et $R_{11}$ représentent, indépendamment les uns des autres, l'hydrogène, F, Cl, Br, un groupe alkyle en $C_1$ à $C_6$, phényle, un groupe $—OCOR_{12}$, un groupe

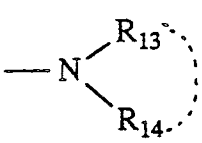

un groupe nitro, cyano, alkoxy en $C_1$ à $C_4$, alkylthio en $C_1$ à $C_4$, hydroxycarbonyle, (alkoxy en $C_1$ à $C_4$)-carbonyle, amino-carbonyloxy, hydroxycarbonyl-(alkyle en $C_1$ à $C_6$), (alkoxy en $C_1$ à $C_4$)-carbonyl-(alkyle en $C_1$ à $C_6$), sulfonyle ou sulfo, et

$R_{12}$ désigne un groupe alkyle en $C_1$ à $C_6$ et $R_{13}$ et $R_{14}$ représentent, indépendamment l'un de l'autre, de l'hydrogène, ou peuvent représenter ensemble ou indépendamment l'un de l'autre un groupe alkyle en $C_1$ à $C_6$ ou un groupe alcanoyle en $C_1$ à $C_6$,

un noyau pentagonal ou hexagonal ayant 1 à 4 hétéro-atomes, identiques ou différents, du groupe des atomes de N, O et S, ou un groupe hydroxycarbonyle, hydroxycarbonyl-(alkyle en $C_1$ à $C_4$), (alkoxy en $C_1$ à $C_6$)-carbonyle, un halogène ou un pseudo-halogène.

2. Composés de β-lactame suivant la revendication 1, dans lesquels le noyau carbocyclique ou hétérocyclique de trois à sept chaînons peut contenir 1 our 2 hétéro-atomes.

3. Procédé de préparation de composés de formule générale (I) suivant la revendication 1

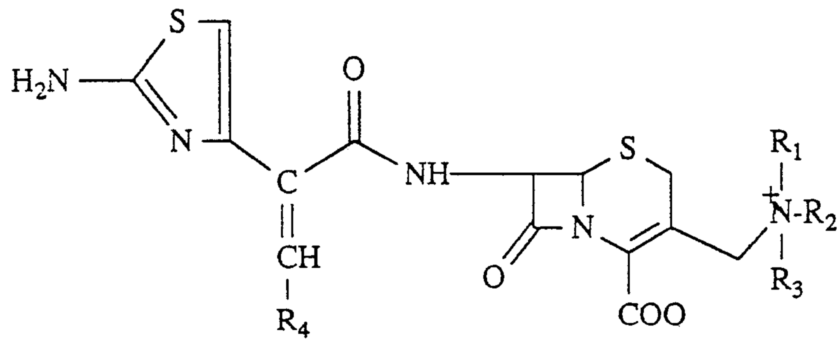

(I)

caractérisé en ce qu'on fait réagir des composés de formule générale (III)

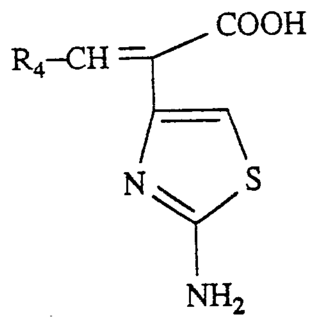

(III)

dans laquelle

$R_4$ a la définition indiquée dans la revendication 1,

dans lesquels le groupe amino peut être protégé ou non protégé, après activation du groupe carboxyle par transformation en un anhydride mixte, notamment avec l'ester éthylique de l'acide chloroformique ou le chlorure d'acide méthane-sulfonique, après transformation en l'halogénure d'acide ou après transformation en un ester activé avec notamment le N-hydroxybenzotriazole et le dicyclohexylcarbodiimide, avec des composés de formule générale (IV)

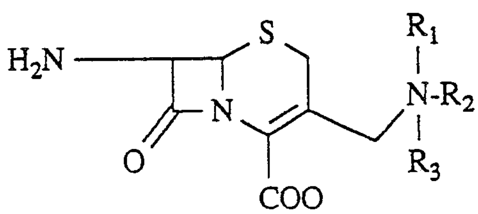

(IV)

dans laquelle

$R_1$, $R_2$ et $R_3$ ont la définition indiquée dans la revendication 1, puis on élimine éventuellement les groupes protecteurs et on prépare les sels désirés ou bien, à partir des sels, les acides libres.

4. Composés de formule générale (IV)

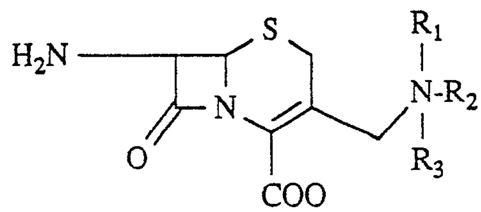

(IV)

dans lesquels

$R_1$, $R_2$ et $R_3$ sont identiques ou sont différents les uns des autres et représentent un reste alkyle en $C_1$ à $C_6$ ou un noyau de trois à sept chaînons, ou bien $R_1$ a la définition indiquée ci-dessus et $R_2$ et $R_3$ forment conjointement avec l'atome $N^+$ un noyau de trois à sept chaînons qui peut être saturé ou non saturé et qui peut contenir 1 ou 2 autres hétéro-atomes qui peuvent être des atomes d'oxygène, d'azote et/ou de soufre.

5. Médicaments contenant au moins un composé suivant l'une des revendications 1 et 2.

6. Utilisation de composés suivant l'une des revendications 1 et 2 pour la préparation de médicaments.

7. Utilisation de composés de β-lactame de formule (I) suivant la revendication 1

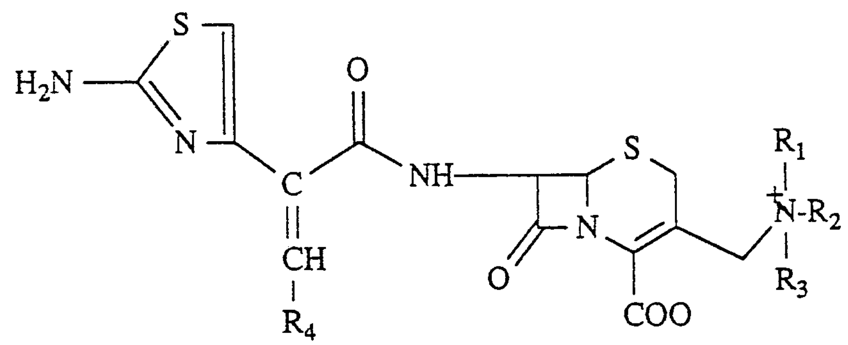

(I)

dans laquelle

$R_1$, $R_2$, $R_3$ et $R_4$ ont la définition indiquée dans la revendication 1, comme activateurs de croissance pour animaux.

## Claims

1. β-Lactam compounds of the general formula (I)

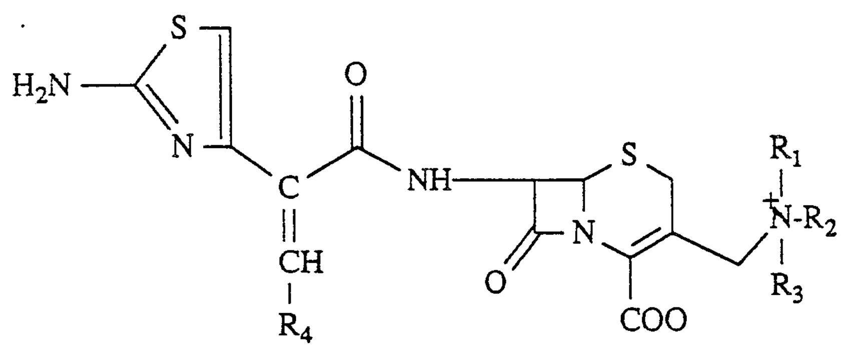

(I)

in which

$R_1$, $R_2$ and $R_3$ are identical to or different from one another and represent a $C_1$—$C_6$-alkyl radical which is optionally substituted by one or two substituents from the group comprising hydroxyl, carboxyl, $C_1$—$C_6$-alkoxycarbonyl, formyl or $C_1$—$C_6$-alkylcarbonyl, carbamoyl, sulpho, cyano, nitro, amino, halogen, $C_1$—$C_6$-alkyl- and dialkylamino, $C_1$—$C_6$-alkylcarbonylamino, $C_1$—$C_6$-alkyloxy, $C_1$—$C_6$-alkylthio, $C_1$—$C_6$-alkylsulphinyl, $C_1$—$C_6$-alkylsulphonyl, phenyl, naphthyl or pyridyl, or represent a 3-to 7-membered ring, or $R_1$ has the above meaning and $R_2$ and $R_3$ form, together with the $N^+$ atom, a 3- to 7-membered carbo- or heterocyclic ring which can be saturated or unsaturated and can contain up to three heteroatoms, which can be oxygen, nitrogen and/or sulphur, and is optionally substituted by one or two substituents from the group comprising $C_1$—$C_6$-alkyl, hydroxyl, hydroxy-$C_1$—$C_6$-alkyl, carboxyl, $C_1$—$C_6$-alkyloxycarbonyl, formyl or $C_1$—$C_6$-alkylcarbonyl, carbamoyl, sulpho, cyano, nitro, amino, halogen, $C_1$—$C_6$-alkyl- and dialkylamino, $C_1$—$C_6$-alkylcarbonylamino, $C_1$—$C_6$-alkyloxy, $C_1$—$C_6$-alkylthio, $C_1$—$C_6$-alkylsulphinyl or $C_1$—$C_6$-alkylsulphonyl, and

$R_4$ denotes hydrogen, an optionally straight-chain or branched alkyl radical which has up to 6 C atoms and can also contain 1 or 2 double bonds and be carbocyclic, an aryl radical of the formula

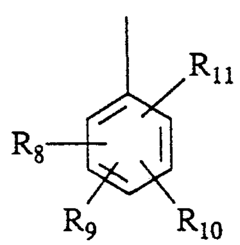

in which

$R_8$, $R_9$ and $R_{11}$ denote, independently of one another, hydrogen, F, Cl, Br, $C_1$—$C_6$-alkyl, phenyl, a group —$OCOR_{12}$, a group

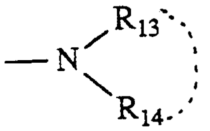

nitro, cyano, $C_1$—$C_4$-alkoxy, $C_1$—$C_4$-alkylthio, hydroxycarbonyl, $C_1$—$C_4$-alkoxycarbonyl, aminocarbonyloxy, hydroxycarbonyl-$C_1$—$C_6$-alkyl, $C_1$—$C_4$-alkoxycarbonyl-$C_1$—$C_6$-alkyl, sulphonyl or sulpho, and in which $R_{12}$ denotes $C_1$—$C_6$-alkyl, and in which $R_{13}$ and $R_{14}$ can be, independently of one another, hydrogen or, together or indepedently of one another, $C_1$—$C_6$-alkyl or $C_1$—$C_6$-alkanoyl, or denotes a 5- or 6-membered ring with 1—4 heteroatoms, identical or different from the group comprising N, O and S, or hydroxycarbonyl, hydroxycarbonyl-$C_1$—$C_4$-alkyl, $C_1$—$C_6$-alkoxycarbonyl, halogen or pseudohalogen.

2. β-Lactam compounds according to Claim 1, in which the 3- to 7-membered carbo- or heterocyclic ring can contain one or two heteroatoms.

3. Process for the preparation of compounds of the general formula (I) according to Claim 1

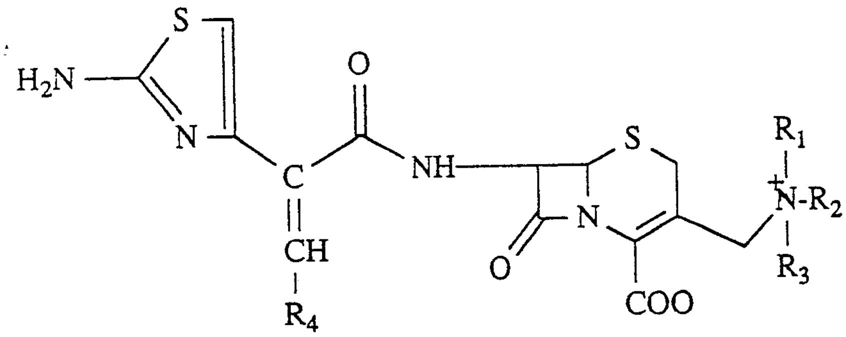

(I)

characterized in that compounds of the general formula (III)

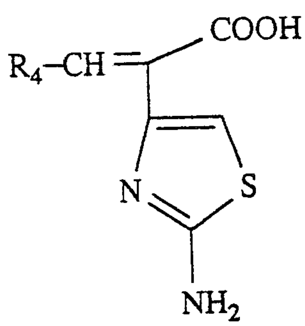

(III)

in which

$R_4$ has the meaning indicated in Claim 1, in which the amino group can be in the protected or unprotected form, is reacted, after activation of the carboxyl group by conversion into a mixed anhydride, in particular with ethyl chloroformate or methanesulphonyl chloride, after conversion into the acid halide or after conversion into an activated ester with, in particular, N-hydroxybenzotriazole and dicyclohexylcarbodiimide, with compounds of the general formula (IV)

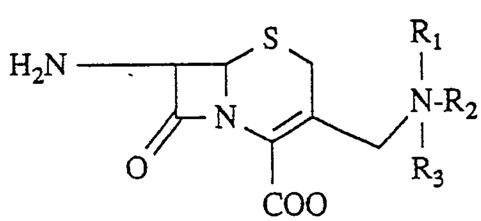

(IV)

in which

$R_1$, $R_2$ and $R_3$ have the meaning mentioned in Claim 1, then, where appropriate, the protecting groups are eliminated, and the desired salts are prepared or the free acids are prepared from salts.

4. Compounds of the general formula (IV)

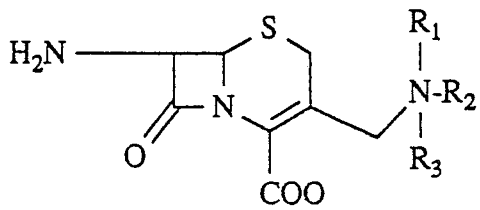

(IV)

in which

$R_1$, $R_2$ and $R_3$ are identical to or different from one another and represent a $C_1$—$C_6$-alkyl radical or a 3- to 7-membered ring, or $R_1$ has the abovementioned meaning and $R_2$ and $R_3$ form, together with the $N^+$ atom, a 3- to 7-membered ring which can be saturated or unsaturated and can contain one or two further heteroatoms, which can be oxygen, nitrogen and/or sulphur.

5. Medicament containing at least one compound according to one of Claims 1 and 2.

6. Use of the compounds according to one of Claims 1 and 2 for preparing medicaments.

7. Use of β-lactam compounds of the formula (I) according to Claim 1

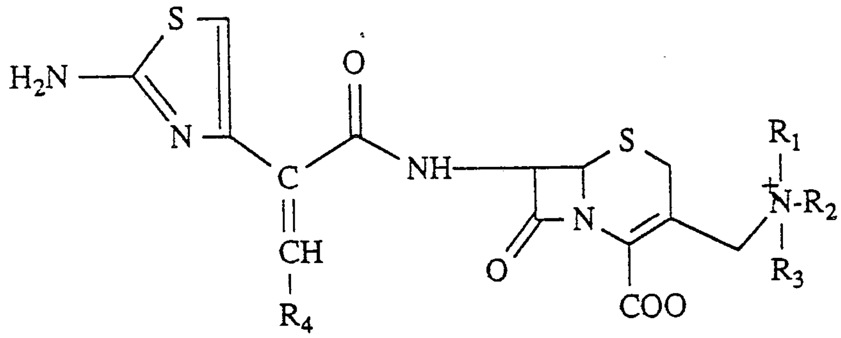

(I)

in which

$R_1$, $R_2$, $R_3$ and $R_4$ have the meaning specified in Claim 1, as growth-promoters for livestock.